# EUROPÄISCHE PATENTSCHRIFT

(11) **EP 2 838 413 B1**
(45) Veröffentlichungstag und Bekanntmachung des Hinweises auf die Patenterteilung: **04.03.2020**
(21) Anmeldenummer: 13732083.4
(22) Anmeldetag: 18.04.2013
(51) Int. Cl.: A61B 1/00, A61B 1/06

(54) **ROTATIONSVORRICHTUNG ZUM ROTIEREN EINES ENDOSKOPS**
ROTATIONAL DEVICE FOR ROTATING AN ENDOSCOPE
DISPOSITIF DE ROTATION D'UN ENDOSCOPE

(30) Priorität: 18.04.2012 DE 102012206413
(43) Veröffentlichungstag der Anmeldung: 25.02.2015
(73) Patentinhaber: Karl Storz SE & Co. KG, 78532 Tuttlingen (DE)
(72) Erfinder: OGINSKI, Stefan, 36041 Fulda (DE); KELP, Martin, 10243 Berlin (DE); ERBER, Felix, 85290 Geisenfeld (DE)
(74) Vertreter: Wimmer, Stephan
(86) Internationale Anmeldenummer: PCT/DE2013/200011
(87) Internationale Veröffentlichungsnummer: WO 2013/156025

(56) Entgegenhaltungen:
- DE-A1- 2 441 222
- JP-A- 2003 159 214
- US-A1- 2010 022 838
- US-A1- 2010 185 212
- US-A1- 2012 065 470

## Beschreibung

Die Erfindung betrifft eine Rotationsvorrichtung zum Rotieren eines Endoskops.

Eine derartige Rotationsvorrichtung umfasst eine rotierbare Baugruppe und eine feststehende Baugruppe. Mit der rotierbaren Baugruppe kann ein Endoskop verbunden werden. Die rotierbare Baugruppe ist relativ zu der feststehenden Baugruppe rotierbar. Eine erste Antriebseinrichtung ist ausgebildet, die rotierbare Baugruppe in eine Drehbewegung um eine Längsachse zu versetzen, um einen Schaft eines mit der rotierbaren Baugruppe verbundenen Endoskops zu rotieren.
Eine Rotationsvorrichtung der hier beschriebenen Art kann insbesondere zum Rotieren eines medizinischen Endoskops verwendet werden, wie es insbesondere in mikroinvasiven Operationen an menschlichen oder tierischen Patienten eingesetzt wird. Ein solches Endoskop weist einen Schaft auf, der einem Operationsort zugeführt wird und unter anderem zum Übertragen von Licht von dem Operationsort hin zu einer Kameravorrichtung dient. Mittels der Kameravorrichtung wird das Licht erfasst und in ein analoges oder digitales Signal gewandelt, das zu einer Anzeigevorrichtung übertragen wird, die den Operationsort abbildet und somit einem Operateur eine visuelle Inspektion des Operationsorts gestattet.

Bei mikroinvasiv durchgeführten Eingriffen, beispielsweise am Abdomen eines Patienten, benötigt ein Operateur in der Regel beide Hände, um Instrumente zu führen und zu bedienen und auf diese Weise den Eingriff durchzuführen. Ein Endoskop zur optischen Erfassung und Inspektion des Operationsorts wird bisher meist von einem Kameraassistenten gehalten, der z.B. hinter oder neben dem Operateur steht. Dies kann nachteilig sein, weil der Bewegungsraum des Operateurs eingeschränkt ist. Außerdem muss der Kameraassistent oft über einen längeren Zeitraum eine ergonomisch unvorteilhafte Körperhaltung einnehmen, so dass es mit zunehmender Operationsdauer aufgrund Ermüdung des Operationsassistenten zu einem Verwackeln des Bildes kommen kann.

Um einen Kameraassistenten überflüssig zu machen, werden heutzutage auch Haltesysteme eingesetzt, mit denen ein Endoskop statisch an einem Operationstisch montiert werden kann. Solche Haltesysteme verwenden Haltearme, die einen relativ einfachen Aufbau aufweisen und universell einsetzbar sind, eine Veränderung der Lage des Endoskops aber nur manuell durch Verstellen des Haltearms ermöglichen.

Anstelle solcher Haltesysteme sind auch mechanische, insbesondere mechatronische Führungssysteme bekannt, die eine motorische Verstellung der Lage eines Endoskops ermöglichen. Ein aus der DE 196 09 034 A1 bekanntes Führungssystem ermöglicht beispielsweise, ein Endoskop sowohl um einen Pivotpunkt, der insbesondere einem Einstechpunkt an einer Bauchdecke eines Patienten oder einer anderen Zugangsöffnung an einem Patienten entspricht zu schwenken, als auch um seine Längsachse zu rotieren.

Dies wird bei dem Führungssystem der DE 196 09 034 A1 dadurch erreicht, dass an einem Gehäuse des Endoskops ein Adapter angebracht wird, der über ein Stirnrad mit einem weiteren Stirnrad einer Antriebsvorrichtung derart gekoppelt ist, dass das Endoskop angetrieben durch die Antriebsvorrichtung in eine Drehbewegung versetzt werden kann. Eine Kameravorrichtung, die Licht aus dem Endoskop empfängt, wird hierbei ortsfest gehalten, indem die Kameravorrichtung über einen Haltebügel an einer die Antriebsvorrichtung haltenden Halterung angeordnet ist. Das Endoskop wird also relativ zu der Kameravorrichtung gedreht.

Das Führungssystem gemäß DE 196 09 034 A1 ermöglicht eine Rotation des Endoskops um seine Längsachse und eine entsprechende Einstellung des Sichtfelds. Beispielsweise bei einem Endoskop, das über eine geeignete Optik (sogenannte "Seitenblickoptik") Licht aus einem Bereich seitlich des Endoskopschafts empfängt, dessen feststehende oder einstellbare Blickrichtung nicht parallel zur Längsachse des Endoskopschafts ist, kann durch Rotation des Endoskops um seine Längsachse das Blickfeld geschwenkt und ein Operationsort in unterschiedlichen Betrachtungsrichtungen betrachtet werden.

Aus der DE 101 41 226 C1 ist eine Endoskophalte- und Transporteinheit bekannt, die eine mit einem Schaft eines Endoskops in Anlage zu bringende Antriebshülse aufweist. Um das Endoskop um seine Längsachse zu rotieren, kann die Antriebshülse angetrieben und um die Längsachse gedreht werden.

Aus der US 5,540,649 ist eine Positioniereinrichtung für medizinische Instrumente bekannt, die ein über eine Antriebsvorrichtung angetriebenes Antriebsrad zum Positionieren eines Endoskops insbesondere entlang seiner Längsachse aufweist.

In US 2012/0065470 A1 ist ein robotisches System für ein Endoskop beschrieben. Das von einer Endoskop-Greif-Anordnung 204 gehaltene Endoskop 100 wird von einer Rotationsanordnung 206 um seine Längsachse rotiert.

In US 2010/0185212 A1 ist ein System zum Positionieren eines Endoskops 7 und eines chirurgischen Instruments beschrieben. Ein Endoskop 7 wird mittels einer Einrichtung 1240 um seine Längsachse rotiert. Eine mit dem Endoskop 7 unmittelbar gekoppelte Kamera 8 wird mittels einer Einrichtung 1270 um ihre Längsachse rotiert.

Durch eine Rotationsvorrichtung zum Rotieren eines Endoskops kann das Sichtfeld, in dem mittels einer Kameravorrichtung Licht aus dem Bereich eines Operationsorts aufgenommen werden kann, erweitert und variiert werden, indem das Endoskop rotiert und dadurch das Sichtfeld um die Längsache des Endoskops geschwenkt wird.

Darüber hinaus sind Endoskope bekannt, die ein oder mehrere Bedienelemente z.B. zum Verstellen einer an einem distalen Ende des Endoskops angeordneten Optik aufweisen und über die beispielsweise ein Sichtfeld variiert, eine Zoomeinstellung angepasst oder eine Fokusstellung verändert werden kann. Solche Bedienelemente können herkömmlich, als Bestandteil eines insgesamt manuell zu bedienenden Endoskops, manuell zu betätigen sein.

Es besteht grundsätzlich ein Bedürfnis nach Führungssystemen für Endoskope, die eine motorisch angetriebene Rotation eines solchen an sich manuell zu bedienenden Endoskops um seine Längsachse und gleichzeitig auch eine einfache Handhabung der Endoskope ermöglichen.

Eine Aufgabe der vorliegenden Erfindung ist es, eine verbesserte Endoskopvorrichtung zur Verfügung zu stellen, die insbesondere eine einfache Handhabung eines ein Bedienelement aufweisenden Endoskops ermöglicht.

Diese Aufgabe wird durch den Gegenstand des unabhängigen Anspruchs gelöst.

Ausführungsformen der vorliegenden Erfindung beruhen auf der Idee, bei einer Rotationsvorrichtung zum Rotieren eines Endoskops, die eine erste Antriebseinrichtung zum Antreiben einer rotierbaren Baugruppe zum Rotieren eines Schafts eines mit der rotierbaren Baugruppe verbundenen Endoskops aufweist, eine zweite Antriebseinrichtung vorzusehen, die ausgebildet ist, ein Bedienelement des mit der rotierbaren Baugruppe verbundenen Endoskops relativ zu dem Schaft des Endoskops zu verstellen, um eine über das Bedienelement zu bedienende Baugruppe des Endoskops zu betätigen.
Die vorliegende Erfindung geht von dem Gedanken aus, bei einer Rotationsvorrichtung mindestens zwei Antriebseinrichtungen vorzusehen, die einerseits zum Rotieren eines mit der Rotationsvorrichtung verbundenen Endoskops und andererseits zum Betätigen eines an dem Endoskop vorgesehenen Bedienelements dienen. Die erste Antriebseinrichtung treibt eine rotierbare Baugruppe an, mit der ein Schaft eines Endoskops koppelbar bzw. lösbar mechanisch verbindbar ist, so dass durch Drehen der rotierbaren Baugruppe das Endoskop um die Längsachse, entlang derer sich der Schaft erstreckt, gedreht werden kann. Die zweite Antriebseinrichtung dient zum Verstellen bzw. Bewegen des an dem Endoskop vorgesehenen Bedienelements, wobei mittels der zweiten Antriebseinrichtung das Bedienelement relativ zu dem Schaft und somit unabhängig von einer Drehbewegung des Schafts verstellt werden kann.
Die erste Antriebseinrichtung kann an der feststehenden Baugruppe und/oder an der rotierbaren Baugruppe angeordnet sein und erzeugt ein Drehmoment zum Rotieren der rotierbaren Baugruppe relativ zu der feststehenden Baugruppe. Die erste Antriebseinrichtung umfasst insbesondere einen Elektromotor, einen Ultraschallmotor, einen Piezomotor oder einen anderen Motor.
Es kann eine einzige zweite Antriebseinrichtung zum Betätigen eines Bedienelements vorgesehen sein. Alternativ können mehrere zweite Antriebseinrichtungen vorgesehen sein, die zur Betätigung mehrerer unterschiedlicher Bedienelemente ausgebildet sind. Die zweite Antriebseinrichtung oder jede der zweiten Antriebseinrichtungen umfasst insbesondere einen Elektromotor, einen Ultraschallmotor, einen Piezomotor oder einen anderen Motor.

Das Bedienelement ist als drehbares Bedienrad ausgebildet, das insbesondere um die Längsachse des Endoskops rotierbar ist. Alternativ kann das Bedienelement als Schieber, Hebel oder dergleichen ausgegestaltet sein.
Die zweite Antriebseinrichtung ist vorzugsweise ausgebildet, durch Betätigen des Bedienelements eine Änderung einer Blickrichtung des Endoskops durch Bewegen eines optischen Elements, z.B. eines Schwenkprismas, eines Spiegels oder einer Linse, oder eine Änderung einer Fokus- oder Zoomeinstellung zu bewirken.
Die Rotationsvorrichtung ist vorzugsweise als modulare Baueinheit ausgeführt und kann zusammen mit einem Endoskop verwendet werden, das an sich für eine manuelle Bedienung ausgelegt ist. Mittels der ersten Antriebseinrichtung, die an einem gewöhnlichen Schaft des Endoskops angreift, kann das Endoskop gedreht werden. Mittels der zweiten Antriebseinrichtung kann ein Bedienelement, das für eine manuell Verstellung vogesehen ist, gedreht oder entlang eines geraden oder gekrümmten Pfads bewegt werden, wobei die zweite Antriebseinrichtung über geeignete Mittel zum Verstellen auf das Bedienelement einwirkt.
Die zweite Antriebseinrichtung kann an der rotierbaren Baugruppe angeordnet sein. In diesem Fall wird bei einem Rotieren des Endoskops mittels der ersten Antriebseinrichtung die zweite Antriebseinrichtung mitbewegt. Mittels der zweiten Antriebseinrichtung kann unabhängig von dem Rotieren des Endoskops mit der ersten Antriebseinrichtung dann das Bedienelement verstellt werden, um eine über das Bedienelement zu bedienende Baugruppe, beispielsweise eine Optik des Endoskops, zu verstellen.
Alternativ kann die zweite Antriebseinrichtung an der feststehenden Baugruppe angeordnet sein. Dies wird weiter unten im Einzelnen erläutert.
Die zweite Antriebseinrichtung ist vorzugsweise ausgebildet, das Bedienelement des Endoskops um die Längsachse zu drehen. Das Bedienelement kann beispielsweise als ringförmiges Element an dem Endoskop angeordnet sein und um die Längsachse des Schafts relativ zu dem Schaft drehbar sein. Zum Verstellen der über das Bedienelement zu bedienenden Baugruppe kann mittels der zweiten Antriebseinrichtung das Bedienelement um die Längsachse, entlang derer sich der Schaft erstreckt, gedreht werden.

Die rotierbare Baugruppe, die durch die erste Antriebseinrichtung angetrieben wird, ist insbesondere über einen Adapter oder eine Klemmeinrichtung drehfest mit dem Endoskop gekoppelt. Über den Adapter oder die Klemmeinrichtung ist die rotierbare Baugruppe somit kraft- bzw. reibschlüssig und/oder formschlüssig drehfest mit dem Endoskop verbunden. Deshalb kann durch Antreiben der rotierbaren Baugruppe mittels der ersten Antriebseinrichtung und durch das resultierende Rotieren der rotierbaren Baugruppe relativ zu der feststehenden Baugruppe das Endoskop um seine Längsachse gedreht werden kann.

Wird die Wirkverbindung der rotierbaren Baugruppe mit dem Endoskop über einen Adapter hergestellt, ist der Adapter vorzugsweise entlang der Längsachse von der rotierbaren Baugruppe entnehmbar, um das Endoskop von der rotierbaren Baugruppe zu lösen, oder entlang der Längsache an die rotierbare Baugruppe ansetzbar, um das Endoskop mit der rotierbaren Baugruppe in drehfeste Wirkverbindung zu bringen. Der Adapter kann hierzu in der Gestalt eines im Wesentlichen rotationssymmetrischen Korbs ausgebildet sein, der zum Ansetzen und Befestigen des Endoskops an der rotierbaren Baugruppe entlang der Längsachse an die rotierbare Baugruppe angesetzt, beispielsweise in eine geeignete Aufnahmeöffnung an der rotierbaren Baugruppe eingesteckt oder eingeführt wird. Der Adapter ist in eingestecktem Zustand drehfest an der rotierbaren Baugruppe festgelegt. Die mechanische Verbindung zwischen Adapter und rotierbarer Baugruppe kann gelöst werden, um den Adapter wiederum entlang der Längsachse außer Eingriff von der rotierbaren Baugruppe zu bringen und somit das Endoskop von der rotierbaren Baugruppe zu lösen.

Der Adapter ist insbesondere aus zwei zueinander bewegbaren Adapterteilen aufgebaut, die ausgebildet sind, das Endoskop drehfest zwischen sich aufzunehmen. Die Adapterteile sind beispielsweise mittels eines Scharniers aneinander angelenkt sein, so dass die Adapterteile relativ zueinander geschwenkt werden können, um den Adapter zu öffnen und das Endoskop in den Adapter einlegen zu können. Im geschlossenen Zustand umgreifen die Adapterteile einen Abschnitt des Endoskops nahe dem proximalen Ende des Endoskop in umfänglicher Richtung vollständig oder im Wesentlichen vollständig. An einem oder an beiden Adapterteilen können ein oder mehrere Formschlussabschnitte vorgesehen sein, die in geschlossenem Zustand des Adapters in Umfangsrichtung um die Längsachse formschlüssig an einem zugeordneten Formschlussabschnitt an dem Schaft des Endoskops oder an einem anderen Teil des Endoskops anliegen, um auf diese Weise das Endoskop kraft- bzw. reibschlüssig und/oder formschlüssig drehfest zu halten.

Indem der Adapter in einem Zustand, in dem er mit der rotierbaren Baugruppe gekoppelt ist, kraft- bzw. reibschlüsig und/oder formschlüssig drehfest und auch axial entlang der Längsachse fest mit der rotierbaren Baugruppe verbunden ist, kann durch Rotation der rotierbaren Baugruppe eine Verstellkraft in schlupffreier Weise auf den Adapter und darüber auf das Endoskop zum Rotieren des Endoskops um seine Längsachse übertragen werden.

Die rotierbare Baugruppe wird zum Rotieren des Endoskops um seine Längsachse durch die erste Antriebseinrichtung angetrieben. Die erste Antriebseinrichtung kann hierzu über ein Getriebe, beispielsweise über ein Reibrad, ein Ritzel, einen Schneckentrieb oder einen Kettentrieb, mit der rotierbaren Baugruppe in Wirkverbindung stehen, wobei das Reibrad oder das Ritzel an einer Antriebswelle der Antriebseinrichtung angeordnet ist und durch Drehen des Reibrads oder des Ritzels eine Verstellkraft in die rotierbare Baugruppe und darüber in das Endoskop eingeleitet werden kann.

Alternativ sind andere Bauformen, beispielsweise eine Ausgestaltung ohne Getriebe, möglich, wobei die erste Antriebseinrichtung insbesondere als Hohlläufermotor, Ultraschallmotor oder Piezomotor ohne Getriebe ausgebildet ist.

Die zweite Antriebseinrichtung, die zum Verstellen des Bedienelements dient, kann dazu ausgestaltet sein, über ein Getriebe, z.B. ein Reibrad, ein Ritzel, einen Schneckentrieb oder einen Kettentrieb, eine Verstellkraft in das Bedienelement einzuleiten. Ist ein Reibrad vorgesehen, kann dieses beispielsweise in direkter Anlage mit dem Bedienelement sein, so dass, angetrieben durch die zweite Antriebseinrichtung, das Reibrad eine Drehbewegung auf das Bedienelement überträgt und darüber das Bedienelement verstellt. Treibt die zweite Antriebseinrichtung ein Ritzel an, so kann dieses beispielsweise auf eine zweite rotierbare Baugruppe einwirken, die über eine Klemmeinrichtung oder einen Adapter mit dem Bedienelement in drehfester Wirkverbindung steht, so dass angetrieben durch das Ritzel die zweite rotierbare Baugruppe gedreht und darüber das Bedienelement verstellt werden kann.

Auch die zweite Antriebseinrichtung kann ohne Getriebeausgebildet sein und beispielsweise einen Hohlläufermotor, einen Ultraschallmotor oder einen Piezomotor aufweisen.

Ist ein Reibrad zur Kraftübertragung auf das Bedienelement vorgesehen, so ist das Reibrad insbesondere an einem Hebelarm eines schwenkbaren Hebels angeordnet, wobei in einer Anlageposition des Hebels das Reibrad in Anlage mit dem Bedienelement des mit der rotierbaren Baugruppe verbundenen Endoskops ist. Das Reibrad ist insbesondere in Richtung zu der Anlageposition elastisch vorgespannt. Der Hebel ist beispielsweise elastisch in Richtung der Anlageposition des Reibrads vorgespannt, indem eine mechanische Feder an dem Hebel angreift und den Hebel mit dem daran angeordneten Reibrad in Richtung der Anlageposition des Reibrads drückt. In der Anlageposition steht das Reibrad somit in reibender Wirkverbindung mit dem Bedienelement bzw. ist mit diesem kraft- bzw. reibschlüssig gekoppelt, so dass durch eine Drehbewegung des Reibrads das Bedienelement angetrieben wird. Durch ein geeignetes Betätigungselement kann der Hebel geschwenkt und darüber das Reibrad aus einer Anlageposition gebracht werden, um die Wirkverbindung zwischen dem Reibrad und dem Bedienelement aufzuheben, so dass das Endoskop ohne Beschädigung der Reibradmechanik von der Rotationsvorrichtung entnommen werden kann.

Das Betätigungselement kann als um die Längsachse drehbares Handrad ausgebildet sein, das drehbar an der rotierbaren Baugruppe angeordnet ist. Ein Drehen des Handrads um einen vorbestimmten Winkel kann ein Bewegen des Reibrads aus der Anlageposition bewirken. Alternativ kann das Betätigungselement als Betätigungshebel ausgestaltet sein, der zum Schwenken des das Reibrad tragenden Hebels betätigt werden kann.

Ist das Betätigungselement als Handrad ausgestaltet, so ist es beispielsweise relativ zur rotierbaren Baugruppe schwenkbar oder um die Längsachse rotierbar an der rotierbaren Baugruppe angeordnet und elastisch beispielsweise mittels einer mechanischen Feder gegenüber der rotierbaren Baugruppe vorgespannt.

Bei einer Rotationsvorrichtung, wie sie hier beschrieben ist, treiben insbesondere die erste Antriebseinrichtung eine erste rotierbare Baugruppe, die mit dem Endoskop verbindbar ist, und die an der feststehenden Baugruppe angeordnete zweite Antriebseinrichtung eine zweite rotierbare Baugruppe, die mit dem Bedienelement des Endoskops koppelbar bzw. in Wirkverbindung zu bringen ist, an. In diesem Fall ist die zweite Antriebseinrichtung, genauso wie die erste Antriebseinrichtung, an der feststehenden Baugruppe angeordnet. Die erste Antriebseinrichtung treibt die erste rotierbare Baugruppe an, um das Endoskop zu drehen. Die zweite Antriebseinrichtung treibt die zweite rotierbare Baugruppe an, um darüber das Bedienelement zu drehen oder in anderer Weise zu verstellen.

Insbesondere sind die erste Antriebseinrichtung und die zweite Antriebseinrichtung zum Drehen des Endoskops einerseits und zum Bewegen bzw. Verstellen des Bedienelements andererseits teilweise oder vollständig unabhängig voneinander. Soll mittels der ersten Antriebseinrichtung das Endoskop gedreht werden, so ist die zweite Antriebseinrichtung für ein Verstellen des Bedienelements um die Längsachse mit gleicher Geschwindigkeit wie die erste Antriebseinrichtung zu betreiben, damit das Bedienelement nicht relativ zu dem Schaft des Endoskops gedreht wird. Besteht bei einem Rotieren des Endoskops mittels der ersten Antriebseinrichtung eine Relativgeschwindigkeit zwischen dem Endoskop und dem Bedienelement, so bewirkt diese Relativgeschwindigkeit ein Verstellen des Bedienelements. Soll das Bedienelement nicht relativ zu dem Schaft verstellt werden, ist die zweite Antriebseinrichtung bei einem Drehen des Endoskops so zu betreiben, dass keine Relativbewegung zwischen dem Bedienelement und dem Schaft des Endoskops auftritt.

Soll das Bedienelement verstellt werden, so wird, wenn der Schaft des Endoskops nicht rotiert wird, die zweite Antriebseinrichtung mit Leistung versorgt, insbesondere bestromt. Soll bei einem Rotieren des Endoskops gleichzeitig auch das Bedienelement verstellt werden, kann eine geeignete Relativbewegung durch eine Geschwindigkeitsdifferenz in den Drehgeschwindigkeiten des Schafts des Endoskops und des Bedienelements bewirkt werden.

Die Antriebseinrichtungen umfassen jeweils z.B. einen Servomotor oder einen geregelten Gleichstrommotor oder einen Synchron- oder Asynchron-Drehstrommotor oder einen Schrittmotor und sind insbesondere elektronisch gesteuert.

Die erste und die zweite rotierbare Baugruppe können jeweils ein um die Längsachse relativ zu der feststehenden Baugruppe drehbares Ringelement aufweisen, das über eine Verzahnung mit einem Ritzel der jeweils zugeordneten Antriebseinrichtung in Eingriff steht und zum Übertragen einer Verstellkraft bzw. eines Drehmoments im einen Fall auf den Schaft und im anderen Fall auf das Bedienelement des Endoskops dient. Die Ringelemente können jeweils über eine Klemmeinrichtung oder einen geeigneten Adapter drehfest mit dem Schaft des Endoskops bzw. dem Bedienelement verbunden sein. Alternativ kann durch das Ringelement wiederum eine Reibradmechanik angetrieben werden, mittels derer eine Verstellkraft auf den Schaft bzw. das Bedienelement übertragen wird.

Bei einer Rotationsvorrichtung, wie sie hier beschrieben ist, ist insbesondere eine Lichtquelle zum Einspeisen oder Einkoppeln von Licht in das Endoskop vorgesehen. Die Lichtquelle kann an der rotierbaren Baugruppe angeordnet sein und im Betrieb mit der rotierbaren Baugruppe rotiert werden. Die Lichtquelle steht dabei über einen oder mehrere Kontakte mit einer entsprechenden Anzahl von Schleifringen an der feststehenden Baugruppe in elektrisch leitfähiger Verbindung, so dass die Lichtquelle über einen elektrischen Anschluss an der feststehenden Baugruppe mit elektrischer Leistung versorgt werden kann. Wenn nur ein Kontakt und ein Schleifring vorgesehen sind, wird ein Stromkreis zur Versorgung der Lichtquelle insbesondere über andere elektrisch leitfähige Bestandteile der Rotationsvorrichtung geschlossen.

Eine Endoskopvorrichtung weist eine Rotationsvorrichtung nach der vorangehend beschriebenen Art und ein an der rotierbaren Baugruppe angeordnetes Endoskop auf. Das Endoskop weist insbesondere einen Schaft und ein Bedienelement zum Ändern einer Blickrichtung des Endoskops durch Bewegen eines optischen Elements, z.B. eines Schwenkprismas, einer Drehlinse oder eines Drehspiegels, oder zum Ändern einer Fokus- oder Zoomeinstellung eines optischen Systems des Endoskops auf. Beispielsweise ist ein an einem von der Rotationsvorrichtung abgewandten distalen Ende angeordnetes, um eine Schwenkachse schwenkbares Schwenkprisma vorgesehen, das mittels des Bedienelements verstellt werden kann. Die Rotationsvorrichtung wirkt über die zweite Antriebseinrichtung auf das Bedienelement ein und ist somit dazu ausgelegt, das Schwenkprisma zu verstellen.

Ein Schwenkprisma ermöglicht ein Schwenken eines Sichtfelds um die dem Schwenkprisma zugeordnete Schwenkachse. Das Schwenken des Sichtfelds ist hierbei nur in einer Ebene, nämlich in der Ebene quer zur Schwenkachse möglich. Durch zusätzliches Rotieren des Endoskops, angetrieben durch die erste Antriebseinrichtung, kann dann die Ebene, in der das Schwenkprisma geschwenkt werden kann, rotiert werden, so dass durch überlagerte Rotation des Endoskops und Schwenkbewegung des Schwenkprismas Licht aus einem großen Raum eingefangen und mittels des Endoskops hin zu einer mit dem Endoskop verbundenen Kameravorrichtung geleitet werden kann. Auf diese Weise ist, z. B. bei einem medizinischen Endoskop, die Inspektion eines Operationsortes in einem großen Bereich möglich, ohne dass hierzu die Lage des Endoskops im Raum an sich (abgesehen von der rotatorischen Stellung des Endoskops) verändert werden muss.

Ein nicht zur Erfindung gehörendes Verfahren zum Rotieren eines Endoskops mittels einer Rotationsvorrichtung ist beschrieben, bei dem ein Endoskop mit einer rotierbaren Baugruppe der Rotationsvorrichtung verbunden wird und eine erste Antriebseinrichtung die rotierbare Baugruppe zum Rotieren eines Schafts des mit der rotierbaren Baugruppe verbundenen Endoskops in eine Drehbewegung um eine Längsachse relativ zu einer feststehenden Baugruppe der Rotationsvorrichtung versetzt. Dabei ist vorgesehen, dass eine zweite Antriebseinrichtung der Rotationsvorrichtung ein Bedienelement des mit der rotierbaren Baugruppe verbundenen Endoskops zur Betätigung einer über das Bedienelement zu bedienenden Baugruppe des Endoskops relativ zu dem Schaft des Endoskops verstellt.

Bei einem Verfahren zum Handhaben eines Endoskops werden das Endoskop mit einer rotierbaren Baugruppe einer Rotationsvorrichtung gekoppelt, die rotierbaren Baugruppe relativ zu einer feststehenden Baugruppe der Rotationsvorrichtung mittels einer ersten Antriebseinrichtung rotiert, um einen Schaft des mit der rotierbaren Baugruppe verbundenen Endoskops in eine Drehbewegung um eine Längsachse des Endoskops zu versetzen, und ein Bedienelement des mit der rotierbaren Baugruppe gekoppelten Endoskops mittels einer zweiten Antriebseinrichtung der Rotationsvorrichtung bewegt, um eine mittels des Bedienelements zu bewegende Baugruppe des Endoskops relativ zu dem Schaft des Endoskops bewegen.

Die vorangehend für die Rotationsvorrichtung beschriebenen Vorteile und vorteilhaften Ausgestaltungen finden analog auch auf das Verfahren Anwendung.
Der der Erfindung zugrunde liegende Gedanke soll nachfolgend anhand der in den Figuren dargestellten Ausführungsbeispiele näher erläutert werden. Es zeigen:
- Figur 1: eine schematische Darstellung einer Endoskopvorrichtung;
- Figur 2: eine schematische Darstellung einer weiteren Endoskopvorrichtung;
- Figur 3: eine schematische Schnittdarstellung einer Rotationsvorrichtung zum Rotieren eines Endoskops;
- Figur 4: eine schematische Schnittdarstellung einer weiteren Rotationsvorrichtung zum Rotieren eines Endoskops;
- Figur 5: eine schematische Schnittdarstellung einer weiteren Rotationsvorrichtung zum Rotieren eines Endoskops;
- Figur 6: eine schematische Schnittdarstellung einer weiteren Rotationsvorrichtung zum Rotieren eines Endoskops;
- Figur 7: eine schematische Schnittdarstellung einer weiteren Rotationsvorrichtung zu Rotieren eines Endoskops;
- Figur 8A: eine schematische axonometrische Darstellung einer weiteren Rotationsvorrichtung zum Rotieren eines Endoskops;
- Figur 8B: eine weitere schematische axonometrische Darstellung der Rotationsvorrichtung aus Figur 8A;
- Figur 9A: eine schematische axonometrische Darstellung eines Adapters der Rotationsvorrichtung aus den Figuren 8A und 8B in geöffnetem Zustand;
- Figur 9B: eine schematische axonometrische Darstellung des Adapters aus Figur 9A in geschlossenem Zustand;
- Figur 10: eine schematische Teilschnittdarstellung der Rotationsvorrichtung aus den Figuren 8A bis 9B;
- Figur 11: eine schematische axonometrische Darstellung einer Reibradmechanik der Rotationsvorrichtung aus den Figuren 8A bis 10;
- Figur 12A: eine weitere schematische Darstellung der Reibradmechanik aus Figur 11 in einem Zustand, in dem die Reibradmechanik nicht in Anlage mit einem Endoskop ist;
- Figur 12B: eine weitere schematische Darstellung der Reibradmechanik aus den Figuren 11 und 12A, in einem Zustand, in dem die Reibradmechanik in Anlage mit einem Endoskop zu bringen ist;
- Figur 13A: eine weitere schematische axonometrische Schnittdarstellung eines Ausschnitts der Rotationsvorrichtung aus den Figuren 8A bis 12B, darstellend die Kopplung des Adapters mit einer rotierbaren Baugruppe der Rotationsvorrichtung;
- Figur 13B: eine weitere schematische axonometrische Schnittdarstellung des in Figur 13A dargestellten Ausschnitts bei aufgehobener Kopplung;
- Figur 14: eine weitere schematische axonometrische Darstellung der Rotationsvorrichtung aus den Figuren 8A bis 13B bei von der rotierbaren Baugruppe getrenntem Adapter; und
- Figur 15: eine schematische Schnittdarstellung einer weiteren Rotationsvorrichtung, die zusätzlich eine Lichtquelle zum Einkoppeln von Licht in ein Endoskop aufweist.

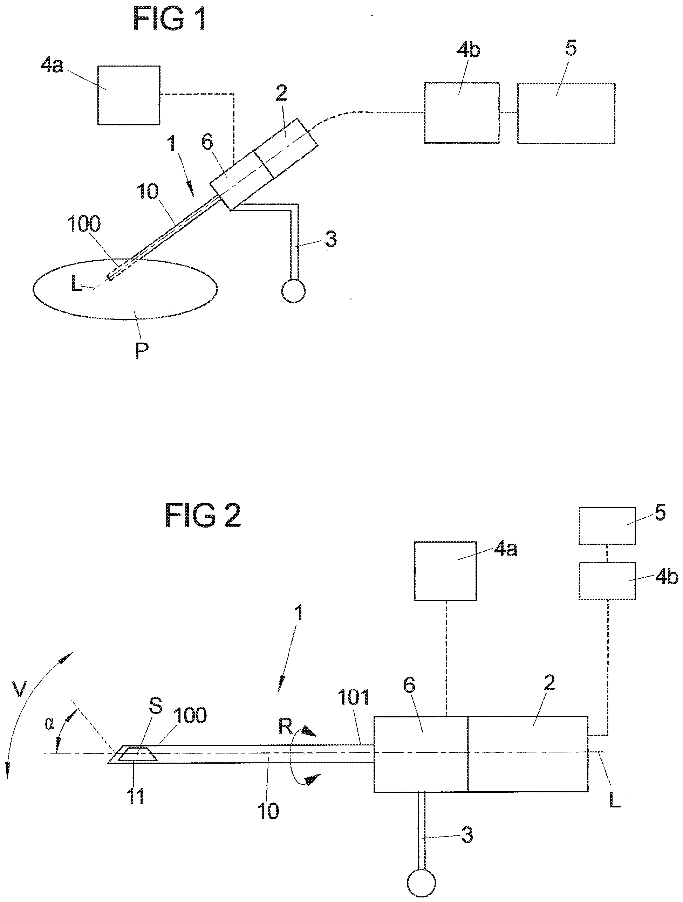

Figur 1 zeigt eine schematische Darstellung einer Endoskopvorrichtung mit einem Endoskop 1 und einer daran angeschlossenen Kameravorrichtung 2, die zur Aufnahme bzw. Erfassung von Bildern aus einem Operationsort an einem Patienten P dient. Das Endoskop 1 weist einen Schaft 10 mit einer Längsachse L auf, dessen distales Ende 100 dem Patienten P zugeführt und in den Patienten P eingeführt ist. Über den Schaft 10 des Endoskops 1 kann Licht von dem Operationsort hin zu der Kameravorrichtung 2 geleitet werden, die das Licht in analoge oder digitale und insbesondere elektrische Signale wandelt und einer Auswerteeinheit 4b zuführt. Die Auswerteeinheit 4b verarbeitet die Signale. Die Signale werden an eine Anzeigevorrichtung 5 in Form eines Monitors übertragen, um Bilder des Operationsorts anzuzeigen.

Eine Halterung 3 ist beispielsweise an einem Operationstisch befestigt. Die Halterung 3 hält eine Rotationsvorrichtung 6, die das Endoskop 1 hält. Die Rotationsvorrichtung 6 ermöglicht eine Rotation des Endoskops 1 um die Längsachse L des Schafts 10 und auf diese Weise eine Variation, insbesondere ein Schwenken, eines Sichtfelds des Endoskops1.

Zur Steuerung der Rotationsvorrichtung 6 dient die Steuereinheit 4a. Die Steuereinheit 4a und die Auswerteeinheit 4b können als separate Einheiten ausgebildet oder abweichend von der Darstellung in Figur 1 gekoppelt sein, um Signale auszutauschen, oder mechanisch und/oder funktionell zu einer Einheit zusammengefasst sein.

Figur 2 zeigt eine schematische Darstellung einer weiteren Endoskopvorrichtung, die in einigen Merkmalen und Eigenschaften der oben anhand der Figur 1 dargestellten Endoskopvorrichtung ähnelt. Nachfolgend sind insbesondere Merkmale und Eigenschaften dargestellt, in denen sich die Endoskopvorrichtung aus Figur 2 von der Endoskopvorrichtung aus Figur 1 unterscheiden.

Bei der in Figur 2 dargestellten Endoskopvorrichtung weist das Endoskop 1 an seinem distalen Ende 100 ein Schwenkprisma 11 auf. Das Schwenkprisma 11 ist, beispielsweise mittels eines in das Endoskop integrierten Motors, mittels einer Schub- oder Druckstange oder auf andere Weise, um eine Schwenkachse S schwenkbar. Die Schwenkachse S ist orthogonal zur Längsachse L des Schafts 10 und orthogonal zur Zeichenebene der Figur 2. Mittels des Schwenkprismas 11 kann Licht aus einem insbesondere kegelförmigen, durch einen Sichtwinkel α beschreibbaren Sichtfeld erfasst werden. Durch Schwenken des Schwenkprismas 11 um die Schwenkachse S (Schwenkbewegung V) kann das Sichtfeld variiert, insbesondere in einer Ebene senkrecht zur Schwenkachse S geschwenkt werden. Durch Rotieren des Schafts 10 des Endoskops 1 mittels der Rotationsvorrichtung 6 kann zusätzlich das Sichtfeld auch um die Längsachse L rotiert werden (Rotationsbewegung R), so dass durch Schwenken des Schwenkprismas 11 einerseits und durch Drehen des Schafts 10 des Endoskops 1 andererseits ein Operationsort in einem großen räumlichen Bereich betrachtet werden kann.

Alternativ zum dargestellten Schwenkprisma kann das Endoskope andere Einrichtungen zum Einstellen bzw. Verändern der Blickrichtung aufweisen, beispielsweise eine Anordnung mit einem oder mehreren rotierbaren oder schwenkbaren Linsen und/oder Spiegeln. Insbesondere kann das Endoskop 1 eine Anordnung zweier reflektierender Flächen, wie sie aus Periskopen bekannt ist, aufweisen. Alternativ kann das distale Ende des Endoskops mechanisch abwinkelbar sein.

Das proximale Ende 101 des Endoskops 1 ist mit der Rotationsvorrichtung 6 und der Kameravorrichtung 2 mechanisch verbunden. Durch die an der Rotationsvorrichtung 6 angreifende Halterung 3 wird die Endoskopvorrichtung in Position gehalten.

Die Kameravorrichtung 2 ist zum Austausch elektrischer oder anderer Signale und zur Übertragung von Leistung mit der Auswerteeinheit 4b gekoppelt. Die Rotationsvorrichtung 6 ist zum Austausch elektrischer oder anderer Signale und zur Übertragung von Leistung mit der Steuereinheit 4a gekoppelt. Die Auswerteeinheit 4b dient dazu, Signale von der Kameravorrichtung 2 zu empfangen und auszuwerten und der Anzeigevorrichtung 5 zuzuführen. Gleichzeitig kann durch die Steuereinheit 4a die Rotationsvorrichtung 6 und mittels dieser sowohl die Rotation des Endoskops 1 als auch das Schwenken des Schwenkprismas 11 gesteuert werden, um auf diese Weise die rotatorische Stellung des Schafts 10 sowie die Stellung des Schwenkprismas 11 einzustellen und zu verändern.

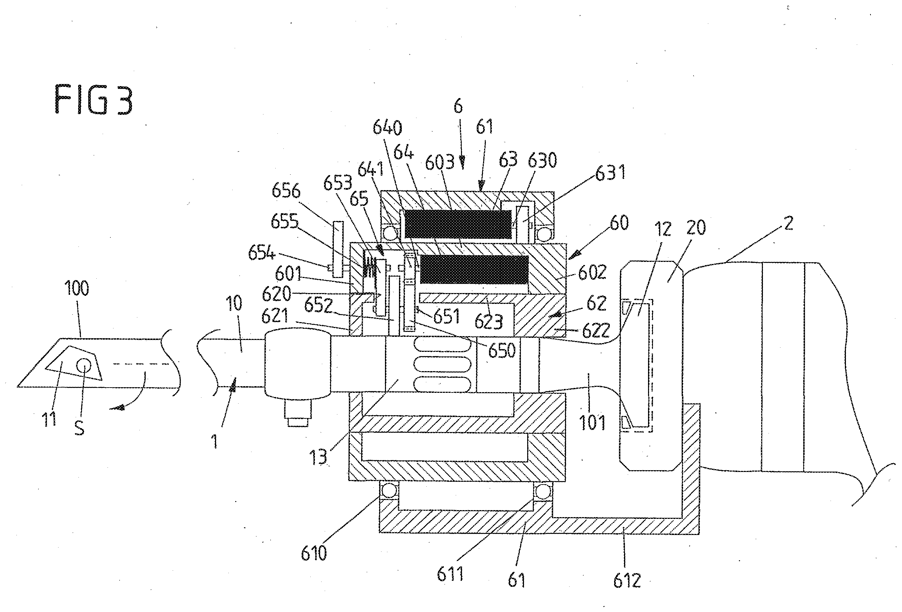

Figur 3 zeigt eine schematische Schnittdarstellung einer Rotationsvorrichtung 6, die in einigen Merkmalen und Eigenschaften der oben anhand der Figuren 1 und 2 dargestellten Rotationsvorrichtung ähnelt. Ferner sind in Figur 3 ein Endoskop 1 und eine Kameravorrichtung 2 in Konturen angedeutet. Die Schnittebene der Figur 3 enthält die Längsachse L des Schafts 10 des Endoskops 1.. Die Rotationsvorrichtung 6, das Endoskop 1 und die Kameravorrichtung 2 können Merkmale und Eigenschaften aufweisen, die den oben anhand der Figuren 1 und 2 dargestellten ähneln. Insbesondere ist die in Figur 3 dargestellte Rotationsvorrichtung 6 eine der oben anhand der Figuren 1 und 2 dargestellten Rotationsvorrichtungen.

Die Rotationsvorrichtung 6 ist ausgebildet, um das Endoskop 1 und die Blickrichtung des Endoskops 1 um die Längsachse L des Endoskops zu rotieren. Ferner ist die Rotationsvorrichtung 6 ausgebildet, um eine Betätigungseinrichtung bzw. ein Bedienelement 13 am proximalen Ende 101 des Endoskops 1 zu betätigen, insbesondere um die Längsachse L zu drehen.

Das Bedienelement 13 ist vorgesehen und ausgebildet, um das am distalen Ende 100 des Endoskops 1 angeordnete und um die Schwenkachse S schwenkbare Schwenkprisma 11 zu schwenken und dadurch ein Sichtfeld des Endoskops 1 zu variieren, insbesondere die Blickrichtung des Endoskops 1 um die Achse S zu schwenken. Das Bedienelement 13 kann hierzu um die Längsachse L des Endoskops 1 verstellt, insbesondere gedreht, werden, wobei durch die Verstellbewegung des Bedienelements 13 das Schwenkprisma 11 um seine Schwenkachse S geschwenkt werden kann.

Die Rotationsvorrichtung 6 weist eine feststehende Baugruppe 61 auf, die mittels eines Stützabschnitts 612 die Kameravorrichtung 2 abstützt bzw hält. Die Rotationsvorrichtung 6 ist beispielsweise durch eine Halterung 3 (siehe Figur 1 und 2) an einem Operationstisch befestigt. Über Lager 610, 611 ist eine rotierbare Baugruppe 60 an der feststehenden Baugruppe 61 um die Längsachse L rotierbar gelagert. Die Lager 610, 611 sind jeweils insbesondere als Kugellager oder anderes Wälzlager oder als Gleitlager ausgebildet. An der feststehenden Baugruppe 61 ist eine Antriebseinrichtung 63 mit einer Antriebswelle 630 angeordnet. Ein Reibrad 631 an der Antriebswelle 630 rollt an einer zylindrischen Mantelfläche 603 der rotierbaren Baugruppe 60 ab, um die rotierbare Baugruppe 60 in eine Drehbewegung um die Längsachse L zu versetzen.

Die rotierbare Baugruppe 60 steht über einen Adapter 62 in drehfester Wirkverbindung mit dem Schaft 10 des Endoskops 1. Der Adapter 62 ist hierzu zwischen Schenkeln oder von nach radial innen ragenden Krägen oder Stützabschnitten 601, 602 der rotierbaren Baugruppe 60 kraft- bzw. reibschlüssig und/oder formschlüssig gehalten und steht über Schenkel oder nach radial innen ragende Krägen oder Stützabschnitte 621, 622 mit dem Schaft 10 des Endoskops 1 in drehfester, z. B. kraft- bzw. reibschlüssiger und/oder formschlüssiger Verbindung.

Die Rotationsvorrichtung 6 ist im Wesentlichen oder zumindest teilweise rotationssymmetrisch aufgebaut. Die feststehende Baugruppe 61 ist insbesondere im Wesentlichen zylindrisch, insbesondere zylindermantelförmig oder kreiszylindermantelförmig, aufgebaut, mit einem in Umfangsrichtung umlaufenden, zylindrischen Ringelement. Die rotierbare Baugruppe 60 besteht im Wesentlichen aus der zylindrischen Mantelfläche 603 und den nach Art von Ringbünden gebildeten, umlaufenden Schenkeln 601, 602.

Wird die rotierbare Baugruppe 60, angetrieben durch die Antriebseinrichtung 63, in eine Drehbewegung um die Längsachse L versetzt, so wird das Endoskop 1 ebenfalls um die Längsachse L gedreht, indem bei einer Drehbewegung der rotierbaren Baugruppe 60 der Schaft 10 des Endoskops 1 über den Adapter 62 mitgenommen wird.

An der rotierbaren Baugruppe 60 ist eine Antriebseinrichtung 64 vorgesehen, die dazu dient, eine Reibradmechanik 65 zum Verstellen des Bedienelements 13 relativ zu dem Schaft 10 anzutreiben. Die Antriebseinrichtung 64 weist eine Antriebswelle 640 auf und steht über ein Ritzel 641 an der Antriebswelle 640 mit einem Zahnrad 650 der Reibradmechanik 65 in Eingriff. Das Zahnrad 650 ist an einer Welle 651 angeordnet und überträgt über die Welle 651 eine Drehbewegung auf ein Reibrad 652, das in reibschlüssiger Verbindung mit dem Bedienelement 13 steht.

Die Welle 651 ist an einem schwenkbaren Hebel 653 so angeordnet, dass sie auf einem Kreisbogen verschiebbar ist. Der Hebel 653 ist über eine Welle 654 mit einem Betätigungselement in Form eines Betätigungshebels 656 gekoppelt. Der Hebel 653 ist über eine Feder 655 in Richtung einer Anlageposition, in der das Reibrad 652 in Anlage mit dem Bedienelement 13 ist, vorgespannt. Mittels des Betätigungshebels 656 kann der Hebel 653 um die Welle 654 geschwenkt werden, um auf diese Weise das Reibrad 652 von dem Bedienelement 13 zu entfernen und somit außer Anlage von dem Bedienelement 13 zu bringen.

Die Reibradmechanik greift in einer betriebsbereiten Stellung bzw. in einem Arbeitsmodus durch eine Öffnung 620 an dem Adapter 62 hindurch. Durch Schwenken des Betätigungshebels 656 kann die Reibradmechanik 65 aus der Öffnung 620 entfernt und so in einen Austauschmodus gebracht werden. Im Austauschmodus können der Adapter 62 mit dem Endoskop 1 entlang der Längsachse L aus der rotierbaren Baugruppe 60 entnommen und dadurch das Endoskop 1 von der Rotationsvorrichtung 6 gelöst bzw. getrennt werden.

Das Endoskop 1 steht in drehfester Verbindung mit der rotierbaren Baugruppe 60 und kann, angetrieben durch die erste Antriebseinrichtung 63 an der feststehenden Baugruppe 61, um seine Längsachse L gedreht werden. Unabhängig davon kann über die zweite Antriebseinrichtung 64 mittels der Reibradmechanik 65 das Bedienelement 13 bewegt werden, um auf diese Weise das Schwenkprisma 11 zu verstellen.

Auf diese Weise kann das Sichtfeld des Endoskops 1 in einem weiten Bereich variiert werden, indem einerseits das Schwenkprisma 11 in einer Ebene orthogonal zur Schwenkachse S verstellt und andererseits diese Ebene durch Rotation des Endoskops 1 um die Längsachse L rotiert werden kann. Auf diese Weise kann, beispielsweise bei Ausgestaltung des Endoskops 1 als medizinisches Endoskop, ein Operationsort in einem großen Sichtbereich inspiziert und überwacht werden.

Die Rotationsvorrichtung 6 ist dafür ausgelegt, ein Endoskop 1, das an sich für eine manuelle Betätigung vorgesehen ist, zu bedienen. Insbesondere kann mittels der zweiten Antriebseinrichtung 64 das für eine manuelle Betätigung vorgesehene Bedienelement 13 rotiert und darüber in motorisierter Weise das Schwenkprisma 11 verstellt werden. Der Adapter 62 kann an die konkrete Bauform eines Endoskops eines bestimmten Herstellers angepasst sein, während andere Teile der Rotationsvorrichtung 6 gleichzeitig für unterschiedliche Bauformen von Endoskope und insbesondere auch für Endoskope unterschiedlicher Hersteller geeignet sein können. Bei einem Wechsel des Endoskops ist also insbesondere nur der Adapter 62 auszutauschen.

Das Endoskop 1 steht mit seinem proximalen Ende 101, das als Okulartrichter 12 gemäß DIN 58105 ausgebildet sein kann, mit einer Kopplungseinrichtung 20 der Kameravorrichtung 2 in Verbindung. Die Kameravorrichtung 2 wird bei einer Rotation des Endoskops 1 lagefest zu der feststehenden Baugruppe 61 gehalten. Die Kopplungseinrichtung 20 der Kameravorrichtung 2 ist dazu ausgelegt, den Okulartrichter 12 in axialer und radialer Richtung zu halten bzw. zu führen, gleichzeitig aber eine Drehbewegung des Okulartrichters 12 relativ zu der Kameravorrichtung 2 zuzulassen.

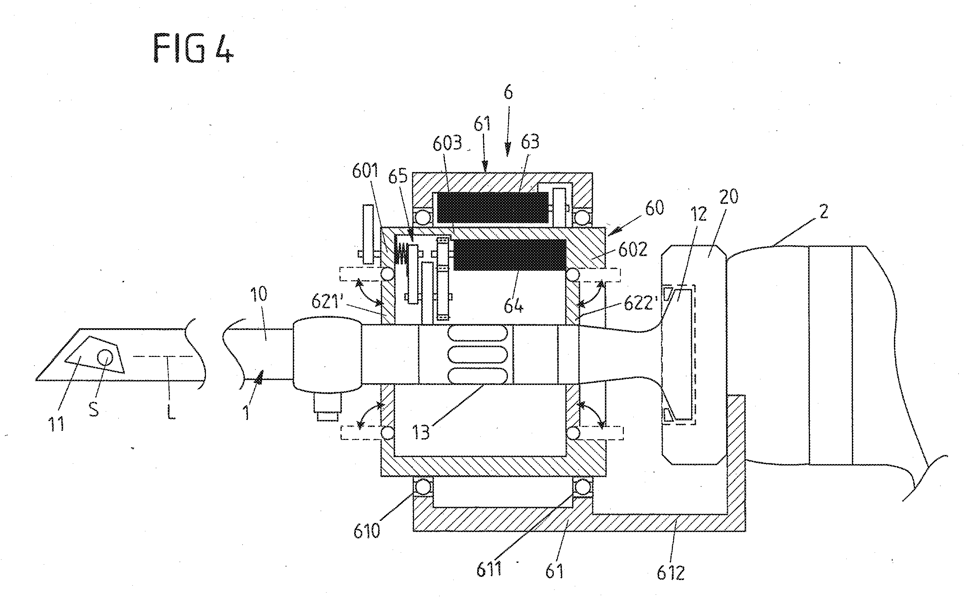

Figur 4 zeigt eine schematische Schnittdarstellung einer Rotationsvorrichtung 6, die in einigen Merkmalen und Eigenschaften den oben anhand der Figuren 1 bis 3 dargestellten Rotationsvorrichtungen ähnelt. Die Art der Darstellung, insbesondere die Schnittebene, entsprechen der von Figur 3. Insbesondere sind auch in Figur 4 ein Endoskop 1 und eine Kameravorrichtung 2 in Konturen angedeutet. Nachfolgend sind insbesondere Merkmale und Eigenschaften beschrieben, in denen sich die Rotationsvorrichtung 6 von den oben anhand der Figuren 1 bis 3 dargestellten Rotationsvorrichtungen unterscheidet.

Die in Figur 4 dargestellte Rotationsvorrichtung 6 unterscheidet sich von den oben anhand der Figuren 1 bis 3 dargestellten Rotationsvorrichtungen insbesondere dadurch, dass kein separater, zusätzlicher Adapter 62 zur Verbindung der rotierbaren Baugruppe 60 mit dem Schaft 10 des Endoskops 1 vorgesehen ist. Stattdessen ist an der rotierbaren Baugruppe 60 eine Klemmeinrichtung 621', 622' in Form von schwenkbar an den Schenkeln 601, 602 der rotierbaren Baugruppe 60 angeordneten Klemmschenkeln vorgesehen. Zum drehfesten Verbinden des Schafts 10 des Endoskops 1 mit der rotierbaren Baugruppe 60 werden die Klemmschenkel 621', 622' in Anlage mit dem Schaft 10 gebracht.

Die Klemmschenkel 621', 622' werden insbesondere durch eine in Figur 4 nicht dargestellte Arretierung und/oder durch eine oder mehrere Federn in den in Figur 4 in durchgezogenen Linien und Schraffur dargestellten Positionen gehalten. Nach Lösen der Arretierung und/oder gegen die Kraft der Feder oder der Federn können die Klemmschenkel 621', 622' in die in gestrichelten Linien dargestellten Positionen gebracht werden, in denen sie nicht am Endoskop 1 anliegen und das Endosko aus der Rotationsvorrichtung 6 entnommen werden kann.

Die zur Anlage am Schaft 10 eines Endoskops 1 bestimmten Flächen der Klemmschenkel 621', 622' sind hinsichtlich ihrer Oberflächeneigenschaften und/oder Oberflächenstruktur derart ausgebildet, dass der Schaft 10 des Endoskops 1 reib- bzw. kraftschlüssig und/oder formschlüssig drehfest mit der rotierbaren Baugruppe 60 der Rotationsvorrichtung 6 verbunden ist.

In weiteren Merkmalen und Eigenschaften entspricht die Rotationsvorrichtung 6 insbesondere der oben anhand der Figur 3 dargestellten Rotationsvorrichtung.

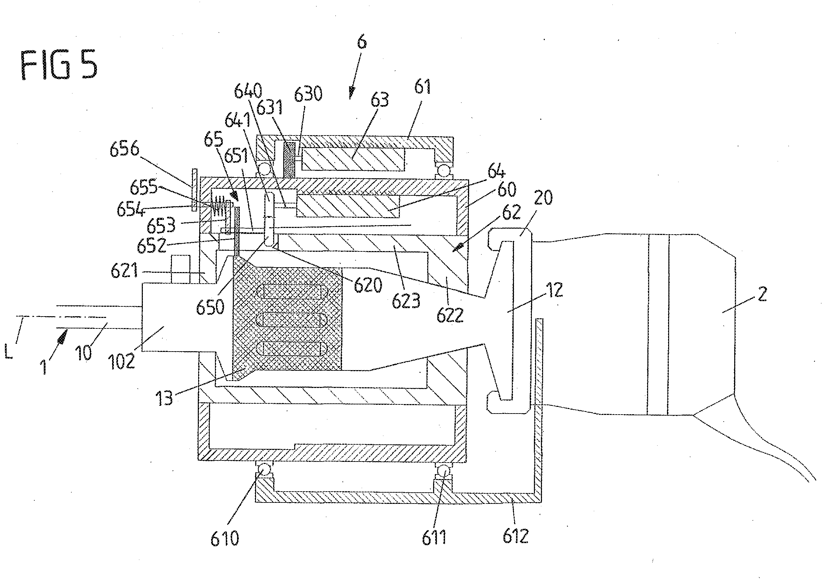

Figur 5 zeigt eine schematische Schnittdarstellung einer Rotationsvorrichtung 6, die in einigen Merkmalen und Eigenschaften den oben anhand der Figuren 1 bis 4 dargestellten Rotationsvorrichtungen ähnelt. Die Art der Darstellung, insbesondere die Schnittebene, entspricht der der Figuren 3 und 4. Insbesondere sind auch in Figur 5 ein Endoskop 1 und eine Kameravorrichtung 2 in Konturen angedeutet, lediglich ein Bedienelement 13 ist durch eine Textur hervorhoben. Nachfolgend sind insbesondere Merkmale und Eigenschaften beschrieben, in denen sich die Rotationsvorrichtung 6 von den oben anhand der Figuren 1 bis 4 dargestellten Rotationsvorrichtungen unterscheidet.

Die in Figur 5 dargestellte Rotationsvorrichtung 6 unterscheidet sich von den oben anhand der Figuren 3 und 4 dargestellten Rotationsvorrichtungen insbesondere dadurch, dass sie für ein anders gestaltetes Endoskop 1 ausgebildet ist. Davon abgesehen ist die Rotationsvorrichtung 6 insbesondere im Wesentlichen bau- und funktionsgleich mit der Rotationsvorrichtung 6 gemäß Figur 3, so dass auf die diesbezüglichen vorangehenden Erläuterungen verwiesen wird.

Das in Figur 5 dargestellte Endoskop 1 weist einen Formschlussabschnitt 102 auf, der hinsichtlich einer Rotation um die Längsachse L des Endoskops 1 formschlüssig mit dem Adapter 62 gekoppelt werden kann, so dass durch eine Drehbewegung des Adapters 62 um die Längsachse L der Schaft 10 des Endoskops 1 mitgenommen und auf diese Weise das Endoskop 1 um seine Längsachse L gedreht wird. Der Formschlussabschnitt weist in einer Schnittebene orthogonal zur Schnittebene der Figur 5 und orthogonal zur Längsachse L beispielsweise eine sechseckige oder eine andere polygonale äußere Kontur auf.

Bei den anhand der Figuren 3 bis 5 dargestellten Rotationsvorrichtungen 6 ist eine erste Antriebseinrichtung 63 an einer feststehenden Baugruppe 61 und eine zweite Antriebseinrichtung 64 an einer rotierbaren Baugruppe 60 angeordnet. Zum Drehen des Endoskops 1 um die Längsachse L wird, insbesondere ausschließlich, die erste Antriebseinrichtung 63 angetrieben, während die zweite Antriebseinrichtung 64 nicht betrieben wird, wenn nicht gleichzeitig das Schwenkprismas 11 mittels des Bedienelements 13 verstellt werden soll. Soll das Bedienelement 13 betätigt werden, so wird die zweite Antriebseinrichtung 64 angetrieben. Soll das Endoskop 1 gedreht und gleichzeitig das Bedienelement 13 betätigt werden, so werden die Antriebseinrichtungen 63, 64 gleichzeitig, aber unabhängig voneinander betrieben.

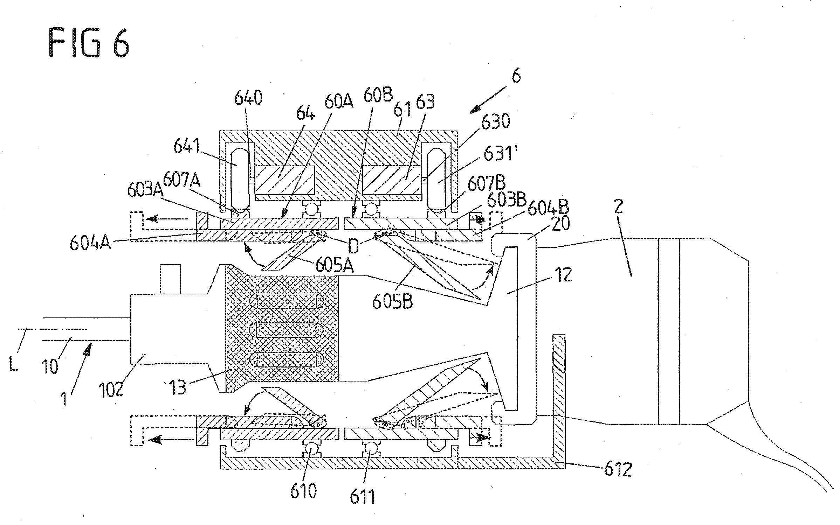

Figur 6 zeigt eine schematische Schnittdarstellung einer Rotationsvorrichtung 6, die in einigen Merkmalen und Eigenschaften den oben anhand der Figuren 1 bis 5 dargestellten Rotationsvorrichtungen ähnelt. Die Art der Darstellung, insbesondere die Schnittebene, entspricht der der Figuren 3 bis 5. Insbesondere sind auch in Figur 6 ein Endoskop 1 und eine Kameravorrichtung 2 in Konturen angedeutet, ein Bedienelement 13 des Endoskops 1 ist durch eine Textur hervorgehoben. Nachfolgend sind insbesondere Merkmale und Eigenschaften beschrieben, in denen sich die Rotationsvorrichtung 6 von den oben anhand der Figuren 1 bis 5 dargestellten Rotationsvorrichtungen unterscheidet.

Bei den anhand der Figuren 3 bis 5 dargestellten Rotationsvorrichtungen sind die erste Antriebseinrichtung 63 an der feststehenden Baugruppe 61 und die zweite Antriebseinrichtung 64 an der rotierbaren Baugruppe 60 angeordnet. Im Gegensatz dazu sind bei der in Figur 6 dargestellten Rotationsvorrichtung 6 sowohl die erste Antriebseinrichtung 63 als auch die zweite Antriebseinrichtung 64 an einer feststehenden Baugruppe 61 angeordnet. Die Rotationsvorrichtung 6 weist zwei rotierbare Baugruppen 60A, 60B auf, von denen eine erste rotierbare Baugruppe 60B durch die erste Antriebseinrichtung 63 und eine zweite rotierbare Baugruppe 60A durch die zweite Antriebseinrichtung 64 in eine Drehbewegung um die Längsachse L des Endoskops 1 versetzt werden kann.

Die erste rotierbare Baugruppe 60B ist mit einem Ringelement 603B ausgebildet, das das Endoskop 1 um die Längsachse L umschließt und über ein Lager 611, insbesondere ein Kugellager oder ein anderes Wälzlager oder ein Gleitlager, an der feststehenden Baugruppe 61 gelagert ist. Die erste Antriebseinrichtung 63 steht über ein an der Antriebswelle 630 der ersten Antriebseinrichtung 63 angeordnetes Ritzel 631' in Eingriff mit einer Verzahnung 607B an einer äußeren Umfangsfläche des Ringelements 603B. Angetrieben durch die erste Antriebseinrichtung 63 rollt das Ritzel 631' an der Verzahnung 607B ab und dreht das Ringelement 603B um die Längsachse L.

Die zweite Antriebseinrichtung 64 weist ebenfalls ein an einer Antriebswelle 640 angeordnetes Ritzel 641 auf, das mit einer Verzahnung 607A an einem Ringelement 603A der zweiten rotierbaren Baugruppe 60A in Eingriff steht. Angetrieben durch die zweite Antriebseinrichtung 64 versetzt das Ritzel 641 das Ringelement 603A in eine Drehbewegung um die Längsachse L.

Die Verzahnungen 607A, 607B laufen um die Ringelemente 603A, 603B um, so dass über den Eingriff mit dem zugeordneten Ritzel 641, 631' die jeweiligen Ringelemente 603A, 603B um die Längsachse L rotiert werden können.

Die erste rotierbare Baugruppe 60B kann über eine Klemmeinrichtung 605B in Form eines oder mehrerer über einen, insbesondere ringförmigen, Schieber 604B zu betätigenden Klemmhebel mit einem Griffstück des Schafts 10 des Endoskops 1 form- und/oder kraftschlüssig gekoppelt werden. Über die Klemmeinrichtung 605B kann die erste rotierbare Baugruppe 60B somit drehfest an dem Schaft 10 festgelegt werden, so dass bei einer Drehbewegung der ersten rotierbaren Baugruppe 60B der Schaft 10 mitgenommen und auf diese Weise das Endoskop 1 um seine Längsachse L gedreht wird.

Die zweite rotierbare Baugruppe 60A kann über eine Klemmeinrichtung 605A in Form eines oder mehrerer über einen, insbesondere ringförmigen Schieber 604A zu betätigenden Klemmhebel mit dem Bedienelement 13 derart form- und/oder kraft- bzw. reibschlüssig gekoppelt werden, dass bei einer Drehbewegung der zweiten rotierbaren Baugruppe 60A das Bedienelement 13 mitgenommen und auf diese Weise das über das Bedienelement 13 zu verstellende Schwenkprisma 11 (vgl. Figuren 2 bis 4) verstellt wird.

Die Klemmhebel 605A, 605B sind jeweils um eine, insbesondere tangential angeordnete, Schwenkachse D an der zugeordneten rotierbaren Baugruppe 60A, 60B schwenkbar angelenkt und z.B. in eine Richtung weg von einer Anlageposition, in der der Klemmhebel 605A, 605B in Anlage mit dem Schaft 10 bzw. dem Bedienelement 13 ist, federelastisch vorgespannt.

Auch bei der in Figur 6 dargestellten Rotationsvorrichtung 6 ist die Kameravorrichtung 2 über einen Stützabschnitt 612 lagefest zu der feststehenden Baugruppe 61 gehalten. Angetrieben durch die erste Antriebseinrichtung 63 wird das Endoskop 1 somit relativ zur Kameravorrichtung 2 rotiert. Die Kopplungseinrichtung 20 der Kameravorrichtung 2 führt das proximale Ende des Endoskops 1, das als Okulartrichter 12 gemäß DIN 58105 ausgebildet sein kann, in axialer und in radialer Richtung, lässt aber eine Drehbewegung relativ zu der Kameravorrichtung 2 zu.

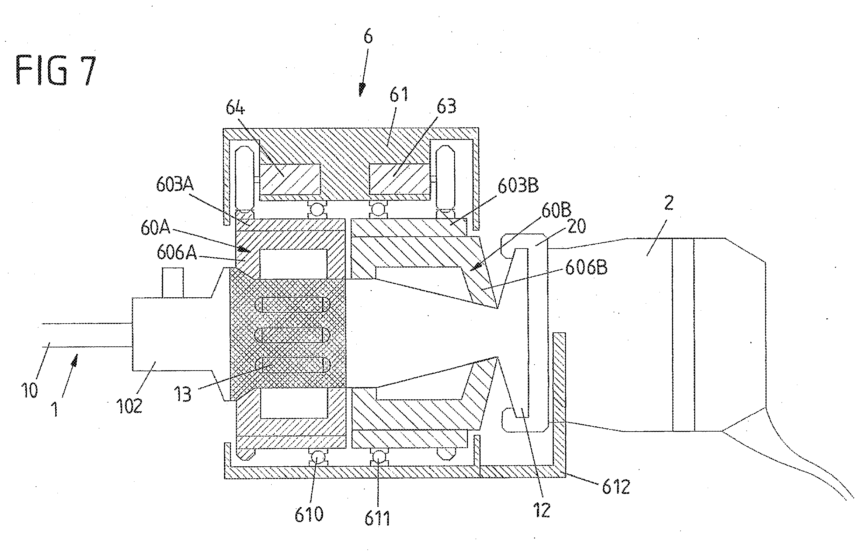

Figur 7 zeigt eine schematische Schnittdarstellung einer Rotationsvorrichtung 6, die in einigen Merkmalen und Eigenschaften den oben anhand der Figuren 1 bis 6 dargestellten Rotationsvorrichtungen ähnelt. Die Art der Darstellung, insbesondere die Schnittebene, entsprechen denen der Figuren 3 bis 6. Insbesondere sind auch in Figur 7 ein Endoskop 1 und eine Kameravorrichtung 2 in Konturen angedeutet, ein Bedienelement 13 des Endoskops 1 ist durch eine Textur hervorgehoben. Nachfolgend sind insbesondere Merkmale und Eigenschaften beschrieben, in denen sich die Rotationsvorrichtung 6 von den oben anhand der Figuren 1 bis 6 dargestellten Rotationsvorrichtungen unterscheidet.

Die in Figur 7 dargestellte Rotationsvorrichtung 6 unterscheidet sich von der anhand der Figur 6 dargestellten Rotationsvorrichtung insbesondere dadurch, dass Adapter 606A, 606B anstelle der Klemmeinrichtungen 605A, 605B vorgesehen sind. Die Adapter 606A, 606B sind vorgesehen und ausgebildet, um je eine Wirkverbindung, insbesondere zur form- und/oder kraftschlüssigen Übertragung von Drehmomenten, zwischen den rotierbaren Baugruppen 60A, 60B einerseits und dem Schaft 10 des Endoskops 1 bzw. dem Bedienelement 13 andererseits zu schaffen. Davon abgesehen ist die Rotationsvorrichtung 6 in ihren Merkmalen und in ihrer Funktionsweise insbesondere ähnlich oder identisch der vorangehend anhand von Figur 6 dargestellten Rotationsvorrichtung 6, so dass auf die vorangehenden Ausführungen verwiesen wird.

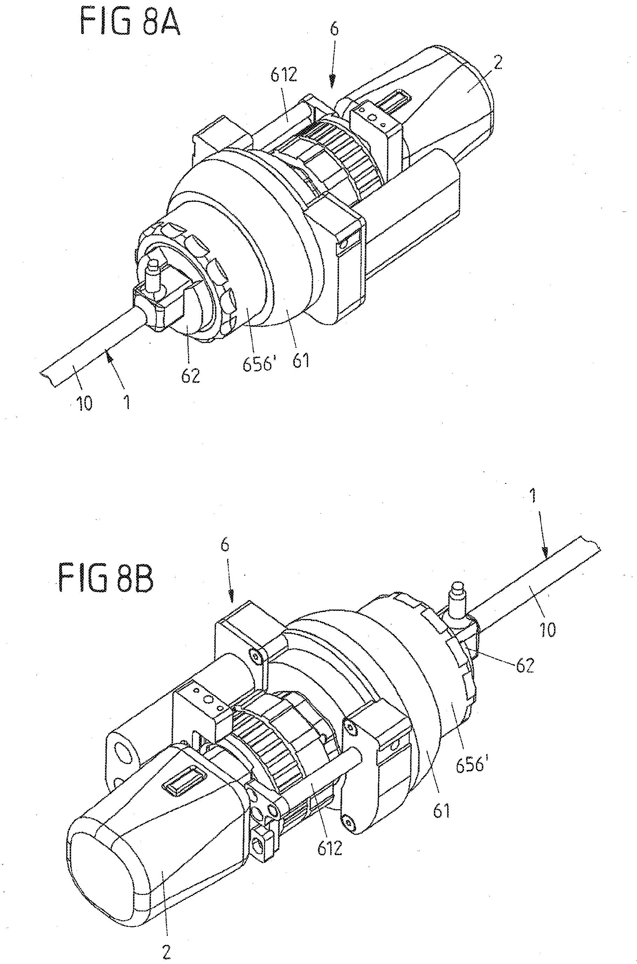

Die Figuren 8A bis 14 zeigen schematische Darstellungen einer Rotationsvorrichtung 6, die in einigen Merkmalen und Eigenschaften den oben anhand der Figuren 1 bis 7 dargestellten Rotationsvorrichtungen ähnelt, insbesondere den oben anhand der Figuren 6 und 7 dargestellten Rotationsvorrichtungen. Nachfolgend sind insbesondere Merkmale und Eigenschaften beschrieben, in denen sich die Rotationsvorrichtung 6 von den oben anhand der Figuren 6 und 7 dargestellten Rotationsvorrichtungen unterscheidet.

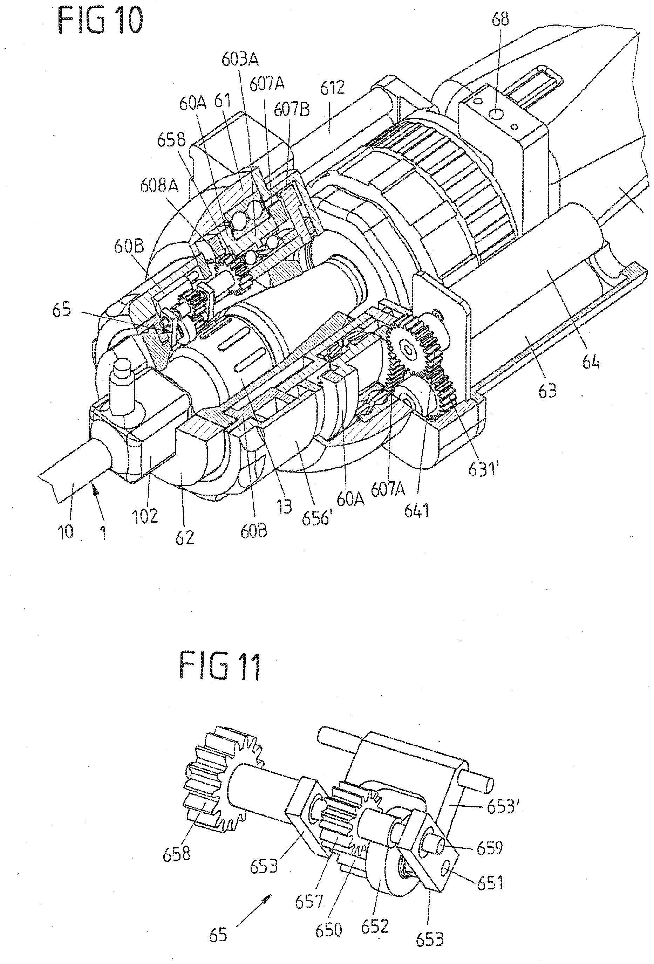

Die in den Figuren 8A bis 14 dargestellte Rotationsvorrichtung 6 weist, wie insbesondere aus der Teilschnittansicht gemäß Figur 10 ersichtlich ist, eine feststehende Baugruppe 61 auf, die über einen Stützabschnitt 612 in lagefester Beziehung zu einer Kameravorrichtung 2 steht. An der feststehenden Baugruppe 61 ist eine rotierbare Baugruppe 60A angeordnet, an der wiederum eine weitere rotierbare Baugruppe 60B drehbar angeordnet ist.

An der feststehenden Baugruppe 61 sind eine erste Antriebseinrichtung 63 und eine zweite Antriebseinrichtung 64 angeordnet. Die erste Antriebseinrichtung 63 wirkt über ein Ritzel 631' auf eine Verzahnung 607B der rotierbaren Baugruppe 60B ein, insbesondere kämmt das Ritzel 631' mit der Verzahnung 607B der rotierbaren Baugruppe 60B. Die zweite Antriebseinrichtung 64 wirkt über ein Ritzel 641 auf eine Verzahnung 607A der rotierbaren Baugruppe 60A ein, insbesondere kämmt dasRitzel 641 mit der Verzahnung 607A der rotierbaren Baugruppe 60A. Die durch die erste Antriebseinrichtung 63 angetriebene rotierbare Baugruppe 60B ist kraft- bzw. reibschlüssig und/oder formschlüssig drehfest verbunden mit einem Adapter 62, der wiederum drehfest (insbesondere formschlüssig drehfest) den Schaft 10 des Endoskops 1 umgreift und auf diese Weise drehfest mit dem Endoskop 1 verbunden ist. Die durch die zweite Antriebseinrichtung 64 angetriebene rotierbare Baugruppe 60A wirkt über eine Verzahnung 608A, die als Innenverzahnung an dem Ringelement 603A der rotierbaren Baugruppe 60A umläuft, auf ein Ritzel 658 einer Reibradmechanik 65 ein. Die Reibradmechanik weist ein Reibrad 652 auf und steht über das Reibrad 652 in reibschlüssiger Verbindung mit einem Bedienelement 13 des Endoskops 1.

Figur 11 zeigt eine gesonderte Ansicht der Reibradmechanik 65. Das Ritzel 658 steht mit der als Innenverzahnung ausgebildeten Verzahnung 608A der rotierbaren Baugruppe 60A (vgl. Figur 10) in Eingriff und wird bei einer Drehbewegung der rotierbaren Baugruppe 60A in eine Drehbewegung versetzt. Das Ritzel 658 treibt eine Welle 659 an, an der ein Zahnrad 657 angeordnet ist, das mit einem Zahnrad 650 an einer Welle 651 in Eingriff steht. An der Welle 651 ist das Reibrad 652 angeordnet derart, dass durch eine Drehbewegung des Zahnrads 650 das Reibrad 652 angetrieben und in eine Drehbewegung versetzt werden kann. Die Welle 651 ist von einem um die Welle 659 schwenkbaren Hebel 653 gehalten und somit auf einem Kreisbogenabschnitt um die Welle 659 bewegbar.Der Hebel 653 bildet zusammen mit einem Hebel 653' einen Kniehebel.

Durch Schwenken des Hebels 653 um die Welle 659 kann das Reibrad 652 in Anlage oder außer Anlage mit dem Bedienelement 13 gebracht werden. Das von der Welle 651 abgewandte Ende des Hebels 653' ist hierbei an einem Handrad 656' (vgl. Figuren 8A, 8B, 10, 13A, 13B, 14) angelenkt, das um die Längsachse L relativ zu der rotierbaren Baugruppe 60B drehbar ist. Die Welle 659 hingegen ist ortsfest an der rotierbaren Baugruppe 60B gelagert. Deshalb kann ein Drehen des Handrads 656' um die Längsachse L relativ zu der rotierbaren Baugruppe 60B die Lage des Kniehebels 653, 653' verändern und dadurch das Reibrad 652 an das Bedienelement 13 angenähern oder von dem Bedienelement 13 entfernen.

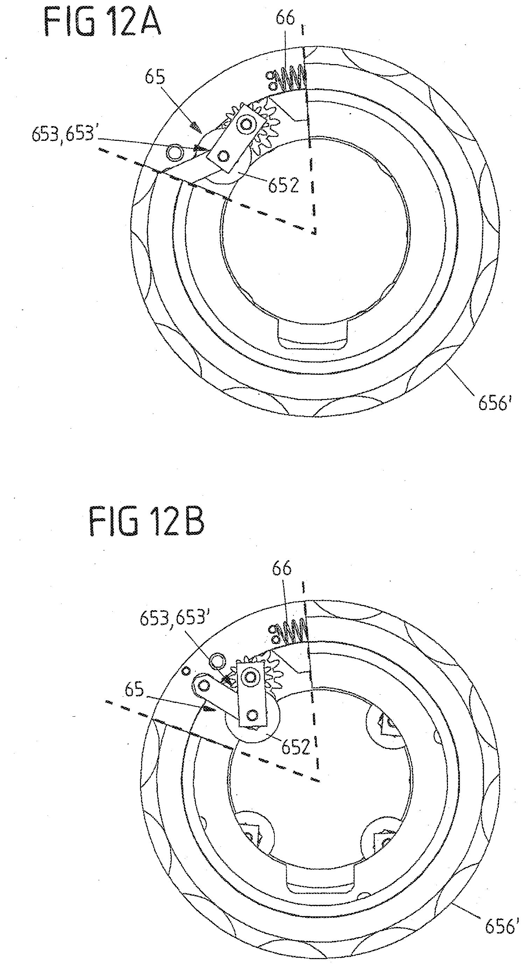

Dies ist in den Figur 12A und 12B dargestellt. Figur 12A zeigt hierbei eine Stellung des Handrads 656', in der das Reibrad 652 von dem Bedienelement 13 (vgl. Figur 10) entfernt ist. Figur 12B zeigt eine Stellung des Handrads 656', in der das Reibrad 652 nach radial innen versetzt und damit in eine Position gebracht ist, in der es, bei an die Rotationsvorrichtung 6 angesetztem Endoskop 1, in reibender Anlage mit dem Bedienelement 13 und somit mit diesem kraft- bzw. reibschlüssig gekoppelt ist.

Die Rotationsvorrichtung 6 weist vier Reibradmechaniken 65 auf, die in Umfangsrichtung versetzt an der rotierbaren Baugruppe 60B angeordnet sind und gleichzeitig mittels des Handrads 656' verstellt werden können.

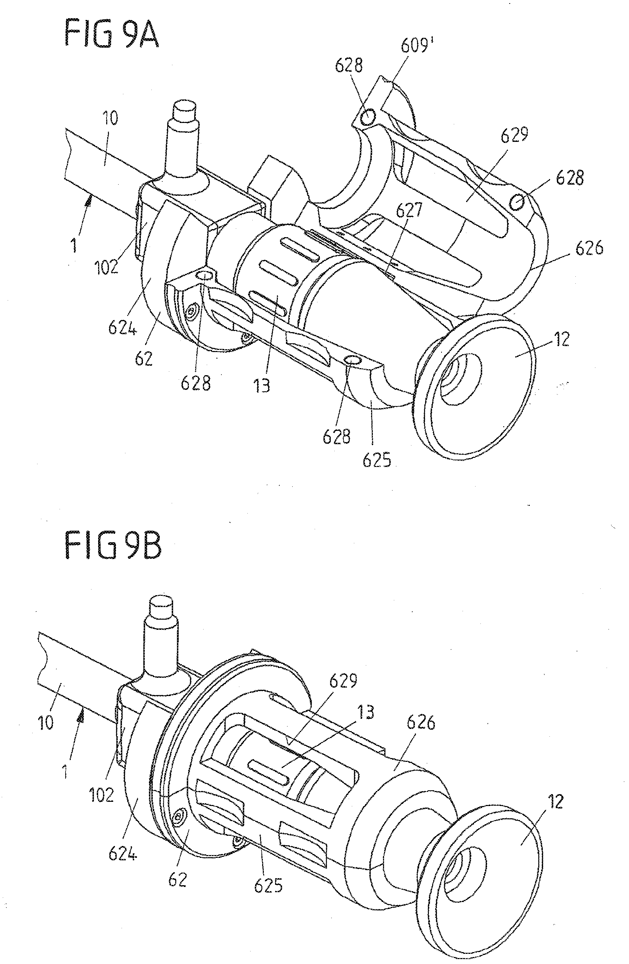

Das Endoskop 1 ist über den Adapter 62 drehfest mit der rotierbaren Baugruppe 60B gekoppelt. Der Adapter 62 weist, wie aus den Darstellungen der Figuren 9A und 9B ersichtlich, eine käfigartige Gestalt mit Aussparungen 629 auf, durch die hindurch die Reibradmechaniken 65 greifen können, um das Reibrad 652 einer jeden Reibradmechanik 65 in reibende Anlage mit dem Bedienelement 13 zu bringen.

Der Adapter 62 weist zwei Adapterteile 625, 626 auf, die über ein Scharnier 627 gelenkig miteinander verbunden sind. In geschlossenem Zustand, dargestellt in Figur 9B, umgreifen die Adapterteile 625, 626 ein Griffstück eines Endoskops 1 umfänglich, wobei an den Adapterteilen 625, 626 Magnete 628 vorgesehen sind, die die Adapterteile 625, 626 in dem geschlossenen Zustand aneinander halten.

An einem Adapterteil 625 ist ein Formschlussabschnitt 624 angeordnet, der in formschlüssigen Eingriff mit einem Formschlussabschnitt 102 des Endoskops 1 zu bringen ist, um auf diese Weise das Endoskop 1 formschlüssig drehfest mit dem Adapter 62 zu koppeln.

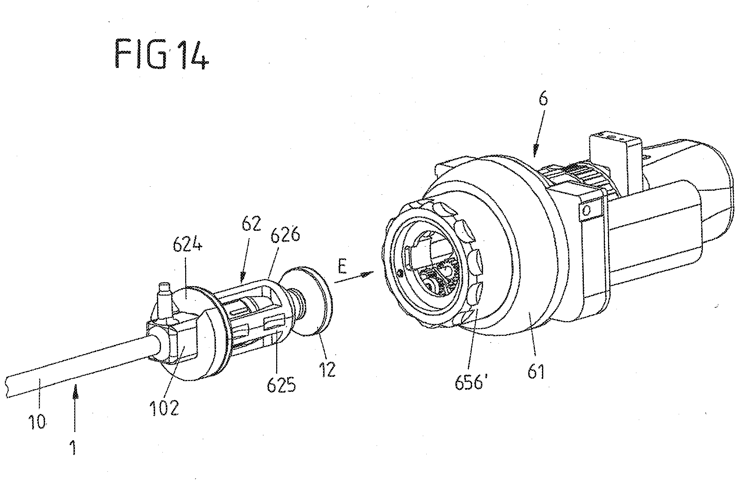

Wie aus Figur 14 ersichtlich, kann das Endoskop 1 zusammen mit den daran angeordneten Adapterteilen 625, 626 in einer Einsteckrichtung E an die Rotationsvorrichtung 6 angesetzt oder in diese eingesetzt werden, um auf diese Weise das Endoskop 1 an der Rotationsvorrichtung 6 lösbar zu befestigen.

Zum Ansetzen des Endoskops 1 zusammen mit dem daran angeordneten Adapter 62 an die Rotationsvorrichtung 6 wird das Handrad 656' betätigt, um die Reibräder 652 der Reibradmechaniken 65 nach radial außen zu bewegen, bis zu dem in Figur 12A dargestellten Zustand. Ist das Endoskop 1 zusammen mit dem Adapter 62 in die durch die rotierbare Baugruppe 60B (vgl. Figur 10) geschaffene Aufnahmeöffnung eingesetzt, wird das Handrad 656' derart verstellt, insbesondere gedreht, dass die Reibräder 652 nach radial innen bewegt werden und somit in Anlage mit dem Bedienelement 13 gelangen, entsprechend dem in Figur 12B dargestellten Zustand.

Das Handrad 656' ist hierbei über Federn 66 (siehe Figur 12A und 12B) in Richtung der Anlageposition vorgespannt, so dass bei einem Loslassen des Handrads 656' die Reibräder 652 selbsttätig nach radial innen und somit in Richtung zu ihrer Anlageposition mit dem Bedienelement 13 bewegt werden.

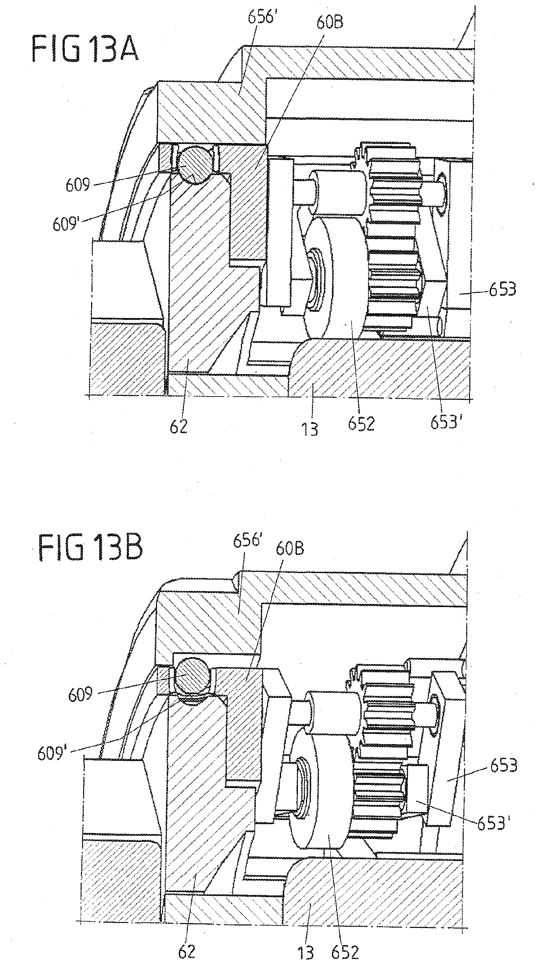

Befinden sich die Reibräder 652 in reibender Anlage mit dem Bedienelement 13, ist der Adapter 62 auch in axialer Richtung relativ zu der rotierbaren Baugruppe 60B mittels einer Axialsicherung 609 arretiert, die in den Figuren 13A und 13B erkennbar ist. Die Axialsicherung 609 umfasst Kugeln, die von dem Handrad 656' in den in Figur 13A gezeigten Positionen gehalten werden, in denen sie in eine Rille 609' an dem Adapter 62 eingreifen. Bei einem Aufheben der reibschlüssigen Verbindung der Reibräder 652 mit dem Bedienelement 13 durch Drehen des Handrads 656' wird auch die Axialsicherung 609 außer Eingriff mit der Rille 609' gebracht, indem die Kugeln nach radial außen und damit aus der Rille 609' heraus bewegbar sind. Danach ist ein Entnehmen des Adapters 62 parallel zur Längsachse L aus der rotierbaren Baugruppe 60B möglich.

Soll bei einer der anhand der Figuren 6, 7, 8A, 8B bis 14 dargestellten Rotationsvorrichtung, bei denen die Antriebseinrichtungen 63, 64 jeweils an der feststehenden Baugruppe angeordnet sind, das Endoskop 1 um seine Längsachse L gedreht werden, dabei aber das Schwenkprisma 11 nicht verstellt werden, so ist erforderlich, dass die erste Antriebseinrichtung 63 und die zweite Antriebseinrichtung 64 für eine gleichartige Bewegung des Schafts 10 und des Bedienelements 13 angetrieben werden, damit keine Relativbewegung zwischen dem Schaft 10 und dem Bedienelement 13 des Endoskops 1 auftritt. Wird eine Relativbewegung zwischen dem Bedienelement 13 und dem Schaft 10 bewirkt, so resultiert ein Verstellen des Schwenkprismas 11 und der Blickrichtung. Eine Ausbildung der Antriebseinrichtungen 63, 64 als Schrittmotoren ermöglicht einen präzisen Antrieb und eine präzise Steuerung der Rotation des Schafts 10 und des Schwenkens des Schwenkprismas 11 mittels des Bedienelements 13.

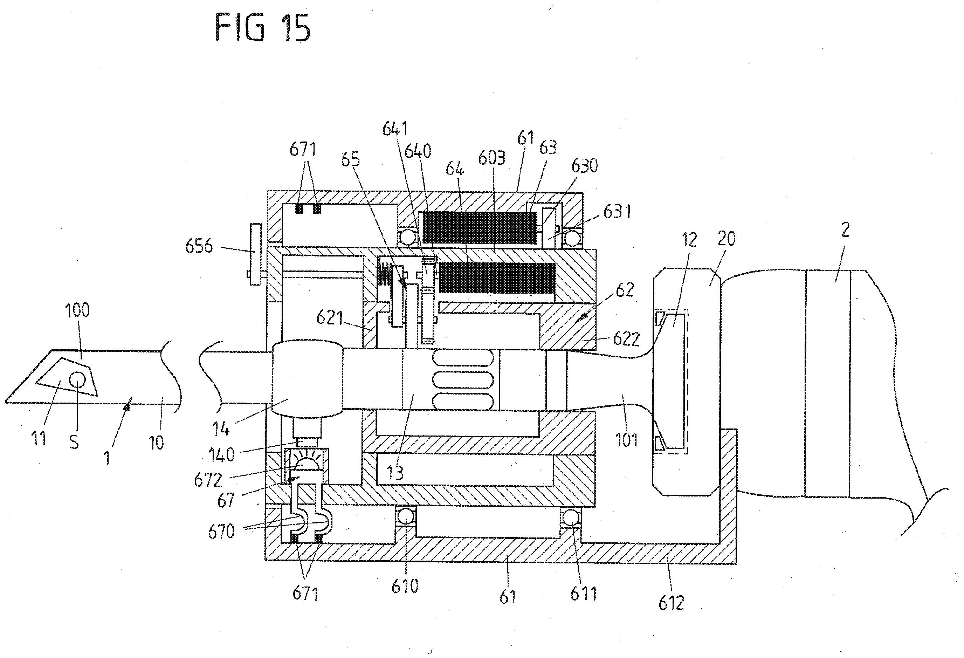

Figur 15 zeigt eine schematische Schnittdarstellung einer Rotationsvorrichtung 6, die in einigen Merkmalen und Eigenschaften den oben anhand der Figuren 1 bis 14 dargestellten Rotationsvorrichtungen ähnelt. Die Art der Darstellung, insbesondere die Schnittebene, entspricht der der Figuren 3 bis 7. Insbesondere sind auch in Figur 15 ein Endoskop 1 und eine Kameravorrichtung 2 in Konturen angedeutet. Nachfolgend sind insbesondere Merkmale und Eigenschaften beschrieben, in denen sich die Rotationsvorrichtung 6 von den oben anhand der Figuren 1 bis 14 dargestellten Rotationsvorrichtungen unterscheidet.

Die in Figur 15 dargestellte Rotationsvorrichtung 6 unterscheidet sich von der anhand der Figur 3 dargestellten Rotationsvorrichtung insbesondere durch eine Lichtquelle 67, die fest an der rotierbaren Baugruppe 60 angeordnet ist, über Kontakte 670 mit Schleifringen 671 an der feststehenden Baugruppe 61 in Kontakt steht und über die Schleifringe 671 und die Kontakte 670 mit elektrischer Leistung versorgt wird. Die Schleifringe 671 sind ringförmig und derart an der feststehenden Baugruppe 61 angeordnet, dass bei einer Rotation der rotierbaren Baugruppe 60 relativ zu der feststehenden Baugruppe 61 die Kontakte 670 schleifend entlang den Schleifringen 671 gleiten und durchgängig in elektrisch leitfähiger Verbindung mit den Schleifringen 671 stehen.

Die Lichtquelle 67 weist eine Lampe oder ein Leuchtmittel 672 auf, die einem Eingang 140 an einer Schnittstelle 14 des Endoskops 1 zugewandt ist und Licht in diesen Eingang 140 einkoppeln kann. Die Lampe umfasst insbesondere eine oder mehrere Leuchtdioden oder Halbleiterlaser oder andere Laser. Bei einer Rotation des Endoskops 1 mit der rotierbaren Baugruppe 60 wird die Lichtquelle 67 - aufgrund der drehfesten Kopplung der rotierbaren Baugruppe 60 mit dem Schaft 10 des Endoskops 1 über den Adapter 62 - gleichförmig mit dem Schaft 10 des Endoskops 1 bewegt. Deshalb bleibt die Lagebeziehung der Lichtquelle 67 zu dem Eingang 140 bei einer Rotation des Endoskops 1 mit der rotierbaren Baugruppe erhalten, und Licht der Lichtquelle 67 kann in den Eingang 140 des Endoskops 1 eingekoppelt werden.

Mittels der Lichtquelle 67 wird eine Beleuchtung eines Operationsortes über das Endoskop 1 möglich. Hierzu wird das in den Eingang 140 eingestrahlte Licht der Lampe 672 durch den Schaft 10 des Endoskops 1 hin zu dem distalen Ende 100 des Endoskops 1 geleitet, so dass das Licht an dem distalen Ende 100 aus dem Schaft 10 austreten und an einen Operationsort gelangen kann. Eine Integration einer Lichtquelle 67 in die rotierbare Baugruppe 60 ermöglicht ein unbeschränktes Rotieren des Endoskops 1 und verbessert somit die Bedienbarkeit der Rotationsvorrichtung 6.

Davon abgesehen ist die in Figur 15 dargestellte Rotationsvorrichtung insbesondere bau- und funktionsgleich mit der anhand der Figur 3 dargestellten Rotationsvorrichtung, so dass auf die obigen Ausführungen verwiesen wird.

Der der Erfindung zugrunde liegende Gedanke ist nicht auf die vorangehend geschilderten Ausführungsbeispiele beschränkt, sondern kann auch bei Endoskop- und Rotationsvorrichtungen mit anderen Merkmalen und Eigenschaften Einsatz finden. So ist eine Rotationsvorrichtung der hier beschriebenen Art insbesondere nicht beschränkt auf den Einsatz bei medizinischen Endoskopen, sondern kann auch bei technischen Endoskopen im Bereich z. B. der technischen Prüfung und Fertigung zum Einsatz kommen.

Die Position und/oder die Orientierung der Rotationsvorrichtung im Raum können erfasst bzw. vermessen werden, indem beispielsweise optische Tracker verwendet werden, die über einen Trackerhalter 68 (siehe z.B. Figur 10) am Gehäuse der Rotationsvorrichtung oder der Kameravorrichtung befestigt sind. Auf diese Weise kann jedem aufgenommenen bzw. erfassten Bild die entsprechende Lage im Raum zugeordnet werden, wenn die momentane rotatorische Stellung des Endoskops und die Stellung des Schwenkprismas bekannt sind. Auf diese Weise ist es möglich, mittels des Endoskops erfasste Bilder mit präoperativ gewonnenen Datensätzes (z. B. aufgenommen unter Verwendung von bildgebenden Verfahren wie CT, MRT oder dergleichen) zu kombinieren, beispielsweise zu überlagern oder zu integrieren.

Zur Erfassung der rotatorischen Stellung der rotierbaren Baugruppe 60 und eines mit dieser gekoppelten Endoskops und zur Erfassung der Stellung eines Schwenkprismas kann jeweils ein mechanischer Endanschlag vorgesehen sein. Insbesondere ist innerhalb der Rotationsvorrichtung ein Endanschlag vorgesehen, welcher es ermöglicht, den Drehwinkel des Endoskops bezüglich der Kameravorrichtung exakt zu bestimmen. Dies kann z. B. über eine Referenzrotation realisiert werden, deren Ablauf in einer Steuerungseinheit gespeichert sein kann und die z. B. beim Anschalten der Versorgungsspannung der Rotationsvorrichtung automatisch initiiert wird. Insbesondere bei Verwendung eines Schrittmotors in der ersten Antriebseinrichtung 63 kann ausgehend von einem Endanschlag jede zukünftig angefahrene Position durch Zählen der Schritte erfasst werden. Der Endanschlag kann konstruktiv beispielsweise derart geschaltet sein, dass er eine Drehung um ca. 380° zulässt, so dass eine volle Umdrehung des Endoskops möglich ist und somit der Sichtbereich nicht eingeschränkt ist.

### Bezugszeichenliste

- 1: Endoskop
- 10: Schaft des Endoskops 1
- 100: Distales Ende des Endoskops 1
- 101: Proximales Ende des Endoskops 1
- 102: Formschlussabschnitt des Endoskops 1
- 11: Schwenkprisma am distalen Ende 100 des Endoskops 1
- 12: Okulartrichter am proximalen Ende 101 des Endoskops 1
- 13: Bedienelement am Endoskop 1
- 14: Schnittstelle
- 140: Eingang
- 2: Kameravorrichtung
- 20: Kopplungseinrichtung der Kameravorrichtung 2
- 3: Halterung zum Halten der Rotationsvorrichtung 6
- 4: Steuereinheit zum Steuern der Rotationsvorrichtung
- 4b: Auswerteeinheit zum Auswerten eines Bildsignals von der Kameravorrichtung 2 und zum Erzeugen eines Bildsignals für die Anzeigevorrichtung 5
- 5: Anzeigevorrichtung zum Anzeigen eines Bilds gesteuert durch die Auswerteeinheit 4b
- 6: Rotationsvorrichtung zum Rotieren des Endoskops 1
- 60, 60A, 60B: Rotierbare Baugruppe der Rotationsvorrichtung 6
- 601, 602: Schenkel oder nach radial innen ragender Kragen oder Stützabschnitt an der rotierbaren Baugruppe 60
- 603: Mantelfläche der rotierbaren Baugruppe 60
- 603A, 603B: Ringelement der rotierbaren Baugruppe 60A, 60B
- 604A, 604B: Schieber am Ringelement 603A, 603B zum Bewegen der Klemmeinrichtungen 605A. 605B
- 605A, 605B: Klemmeinrichtung (Klemmelement)
- 606A, 606B: Adapter
- 607A, 607B: Verzahnung am Ringelement 603A, 603B
- 608A: Verzahnung
- 609: Axialsicherung
- 609': Rille
- 61: Feststehende Baugruppe der Rotationsvorrichtung 6
- 610, 611: Lager zwischen der feststehenden Baugruppe 61 und der
- 612: rotierbaren Baugruppe 60 Stützabschnitt an der feststehenden Baugruppe 61 zum Halten einer Kameravorrichtung 2
- 62: Adapter der Rotationsvorrichtung 6
- 620: Öffnung am Adapter 62
- 621, 622: Schenkel oder nach radial innen ragender Kragen oder Stützabschnitt am Adapter 62
- 621', 622': Klemmeinrichtung (Klemmschenkel)
- 623: Mantelfläche
- 624: Formschlussabschnitt
- 625, 626: Adapterteil
- 627: Scharnier
- 628: Magnet
- 629: Aussparung
- 63: Antriebseinrichtung der Rotationsvorrichtung 6 zum Rotieren der rotierbaren Baugruppe 60
- 630: Antriebswelle der Antriebseinrichtung 63
- 631: Reibrad an der Antriebswelle 630 der Antriebseinrichtung 63
- 631': Ritzel an der ersten Antriebseinrichtung 63
- 64: Antriebseinrichtung der Rotationsvorrichtung 6 zum Betätigen des Bedienelements 13
- 640: Antriebswelle der Antriebseinrichtung 64
- 641: Ritzel an der Antriebswelle 640 der Antriebseinrichtung 64
- 65: Reibradmechanik der Rotationsvorrichtung 6 zum Verstellen des Bedienelements 13
- 650: Zahnrad auf der Welle 651
- 651: Welle am Hebel 653 für das Zahrad 650 und das Reibrad 652
- 652: Reibrad auf der Welle 651
- 653: Hebel, um die Welle 659 schwenkbar
- 653': Hebel zwischen Welle 651 und Handrad 656'
- 654: Welle, um den der Hebel 653 schwenkbar ist
- 655: Feder an der Welle 654
- 656: Betätigungshebel an der Welle 654
- 656': Handrad
- 657: Zahnrad auf der Welle 659
- 658: Ritzel auf der Welle 659
- 659: Welle für das Zahnrad 657 und das Ritzel 658
- 66: Feder für das Handrad 656'
- 67: Lichtquelle der Rotationsvorrichtung 6
- 670: Kontakt der Lichtquelle 67 zur elektrischen Leistungsversorgung der Lampe 672
- 671: Schleifring der Lichtquelle 67 zur elektrischen Leistungsversorgung der Lampe 672
- 672: Lampe der Lichtquelle 67
- 68: Trackerhalter an der Rotationsvorrichtung 6
- α: Sichtwinkel des Endoskops 1
- D: Schwenkachse der Klemmeinrichtung 605A, 605B
- E: Einsteckrichtung des Endoskops 1 und des Adapters 62 in die Rotationsvorrichtung 6
- L: Längsachse des Endoskops 1 und Rotationsachse der rotierbaren Baugruppen 60, 60A, 60B, der Ringelemente 603A, 603B, des Adapters 62 und des Handrads 656'
- P: Patient
- R: Rotationsbewegung des Schafts 10 des Endoskops 1 um die Längsachse L
- S: Schwenkachse des Schwenkprismas 11
- V: Schwenkbewegung der Blickrichtung

## Patentansprüche

1. Endoskopvorrichtung mit:
einem Endoskop (1), das einen Schaft (10) und ein um die Längsachse (L) des Endoskops (1) rotierbares Bedienrad (13) zum Bewegen einer bewegbaren Baugruppe (11) des Endoskops (1) zum Ändern einer Blickrichtung des Endoskops (1) durch Bewegen eines optischen Elements (11) oder zum Ändern einer Fokus- oder Zoomeinstellung eines optischen Systems des Endoskops (1) aufweist; und
einer Rotationsvorrichtung (6) zum Rotieren des Endoskops (1), wobei die Rotationsvorrichtung (6) umfasst:
eine rotierbare Baugruppe (60), mit der das Endoskop (1) verbindbar ist;
eine feststehende Baugruppe (61), relativ zu der die rotierbare Baugruppe (60) rotierbar ist;
eine erste Antriebseinrichtung (63) zum Rotieren der rotierbaren Baugruppe (60) relativ zu der feststehenden Baugruppe (61), um den Schaft (10) des mit der rotierbaren Baugruppe (60) verbundenen Endoskops (1) in eine Drehbewegung um die Längsachse (L) des Endoskops (1) zu versetzen;
eine zweite Antriebseinrichtung (64) zum Rotieren des um die Längsachse des Endoskops (1) rotierbaren Bedienrads (13) des mit der rotierbaren Baugruppe (60) verbundenen Endoskops (1) relativ zu dem Schaft (10) des Endoskops (1), um eine mittels des Bedienrads (13) bewegbare Baugruppe (11) des Endoskops (1) zu bewegen, wobei die zweite Antriebseinrichtung (64) ausgebildet ist, das Bedienrad (13) des Endoskops (1) um die Längsachse (L) des Endoskops (1) zu drehen.

2. Endoskopvorrichtung nach Anspruch 1, bei der die zweite Antriebseinrichtung (64) ausgebildet ist, durch Betätigen des Bedienrads (13) eine Änderung einer Blickrichtung des Endoskops (1) durch Bewegen eines optischen Elements (11) oder eine Änderung einer Fokus- oder Zoomeinstellung eines optischen Systems des Endoskops (1) zu bewirken.

3. Endoskopvorrichtung nach Anspruch 1 oder 2, bei der die zweite Antriebseinrichtung (64) an der rotierbaren Baugruppe (60) oder an der feststehenden Baugruppe (61) angeordnet ist.

4. Endoskopvorrichtung nach einem der vorangehenden Ansprüche, wobei die Rotationsvorrichtung (6) ferner umfasst:
einen Adapter (62, 606B) oder einer Klemmeinrichtung (621', 622', 605B) zur drehfesten Kopplung der rotierbaren Baugruppe (60) mit dem Endoskop (1).

5. Endoskopvorrichtung nach dem vorangehenden Anspruch, bei der der Adapter (62) entlang der Längsachse (L) von der rotierbaren Baugruppe (60) entnehmbar ist, um das Endoskop (1) von der rotierbaren Baugruppe (60) zu trennen, oder entlang der Längsachse (L) an die rotierbare Baugruppe (60) ansetzbar ist, um das Endoskop (1) mit der rotierbaren Baugruppe (60) mechanisch zu koppeln.

6. Endoskopvorrichtung nach Anspruch 4 oder 5, bei der der Adapter (62) zwei relativ zueinander bewegbare Adapterteile (625, 626) aufweist, die ausgebildet sind, das Endoskop (1) drehfest zwischen sich aufzunehmen.

7. Endoskopvorrichtung nach einem der Ansprüche 4 bis 6, bei der der Adapter (62) in einem mit der rotierbaren Baugruppe (60) verbundenen Zustand in einer zur Übertragung eines Drehmoments um die Längsachse (L) und zur Übertragung einer Kraft parallel zur Längsachse (L) geeigneten Weise mit der rotierbaren Baugruppe (60) gekoppelt ist.

8. Endoskopvorrichtung nach einem der vorangehenden Ansprüche, bei der die Rotationsvorrichtung (6) ferner umfasst:
eine Übertragungseinrichtung (641, 652), die mit der zweiten Antriebseinrichtung (64) gekoppelt ist, um angetrieben durch die zweite Antriebseinrichtung (64) eine Verstellkraft auf das Bedienrad (13) auszuüben, wobei die Übertragungseinrichtung ein Reibrad (652) umfasst, das an einem Hebelarm eines schwenkbaren Hebels (653) angeordnet ist, wobei in einer Anlageposition des Hebels (653) das Reibrad (652) in Anlage mit dem Bedienrad (13) des mit der rotierbaren Baugruppe (60) verbundenen Endoskops (1) ist, und wobei der Hebel (653) mittels eines Betätigungselements (656, 656') schwenkbar ist.

9. Endoskopvorrichtung nach dem vorangehenden Anspruch, bei der das Betätigungselement ein um die Längsachse (L) drehbares Handrad (656') umfasst, das drehbar an der rotierbaren Baugruppe (60) angeordnet und zum Bewegen des Reibrads (652) aus der Anlageposition bewegbar ist.

10. Endoskopvorrichtung nach einem der vorangehenden Ansprüche, bei der die erste Antriebseinrichtung (63) zum Antrieb einer ersten rotierbaren Baugruppe (60B), die mit dem Endoskop (1) mechanisch verbindbar ist, ausgebildet ist, und bei der die an der feststehenden Baugruppe (61) angeordnete zweite Antriebseinrichtung (64) zum Antrieb einer zweiten rotierbaren Baugruppe (60A), die mit dem Bedienrad (13) des Endoskops (1) koppelbar ist, ausgebildet ist.

11. Endoskopvorrichtung nach dem vorangehenden Anspruch, bei der die erste und die zweite rotierbare Baugruppe (60A, 60B) jeweils ein um die Längsachse (L) relativ zu der feststehenden Baugruppe (61) drehbares Ringelement (603A, 603B) aufweisen, das über eine Verzahnung (617A, 617B) mit einem Ritzel (631', 641) der jeweils zugeordneten Antriebseinrichtung (63, 64) in Eingriff steht und zum Übertragen eines Drehmoments auf das Endoskop (1) oder auf das Bedienrad (13) des Endoskops (1) ausgebildet ist.

12. Endoskopvorrichtung nach einem der vorangehenden Ansprüche, bei der die Rotationsvorrichtung (6) ferner eine Lichtquelle (15) zum Erzeugen und Einspeisen von Licht in das Endoskop (1) umfasst.

13. Endoskopvorrichtung nach dem vorangehenden Anspruch, bei der die Lichtquelle (15) an der rotierbaren Baugruppe (60) angeordnet und ausgebildet ist, um mit der rotierbaren Baugruppe (60) zu rotieren.

## Claims

1. Endoscope device comprising:
an endoscope (1) comprising a shaft (10) and an operating wheel (13) rotatable about the longitudinal axis (L) of the endoscope (1) for moving a movable assembly (11) of the endoscope (1) in order to change a direction of view of the endoscope (1) by moving an optical element (11) or in order to change a focus or zoom setting of an optical system of the endoscope (1); and
a rotational device (6) for rotating the endoscope (1), wherein the rotational device (6) comprises:
a rotatable assembly (60), to which the endoscope (1) is connectable;
a stationary assembly (61), relative to which the rotatable assembly (60) is rotatable;
a first drive means (63) for rotating the rotatable assembly (60) relative to the stationary assembly (61) in order to put a shaft (10) of the endoscope (1) connected to the rotatable assembly (60) into rotational motion about the longitudinal axis (L) of the endoscope (1);
a second drive means (64) for rotating the operating wheel (13) of the endoscope (1) connected to the rotatable assembly (60) relative to the shaft (10) of the endoscope (1) in order to move an assembly (11) of the endoscope (1), which assembly is movable by means of the operating wheel (13), which operating wheel (13) is rotatable about the longitudinal axis of the endoscope (1), wherein the second drive means (64 is configured to rotate the operating wheel (13) of the endoscope (1) about the longitudinal axis (L) of the endoscope (1).

2. Endoscope device accorrding to claim 1, wherein the second drive means (64) is embodied to, by way of actuating the operating wheel (13), bring about a change in a direction of view of the endoscope (1) by moving an optical element (11), or a change in a focus or zoom setting of the optical system of the endoscope (1).

3. Endoscope device according to claim 1 or 2, wherein the second drive means (64) is arranged at the rotatable assembly (60) or at the stationary assembly (61).

4. Endoscope device according to one of the preceding claims, the rotational device (6) furthermore comprising:
an adapter (62, 606B) or a clamping means (621', 622', 605B) for rotationally secured coupling of the rotatable assembly (60) to the endoscope (1).

5. Endoscope device according to the preceding claim, wherein the adapter (62) is removable from the rotatable assembly (60) along the longitudinal axis (L) in order to separate the endoscope (1) from the rotatable assembly (60) or joinable to the rotatable assembly (60) along the longitudinal axis (L) in order to mechanically couple the endoscope (1) to the rotatable assembly (60).

6. Endoscope device according to claim 4 or 5, wherein the adapter (62) comprises two adapter parts (625, 626) movable relative to one another, which are embodied to hold the endoscope (1) between them in a rotationally secured manner.

7. Endoscope device according to one of the claims 4 to 6, wherein the adapter (62), in a state connected to the rotatable assembly (60), is coupled to the rotatable assembly (60) in a manner suitable for transmitting a torque about the longitudinal axis (L) and for transmitting a force parallel to the longitudinal axis (L).

8. Endoscope device according to one of the preceding claims, the rotational device (6) furthermore comprising:
a transmission means (641, 652) coupled to the second drive means (64) in order to exert, driven by the second drive means (64), an adjustment force on the operating wheel (13), wherein the transmission means comprises a friction wheel (652) arranged at a lever arm of a pivotable lever (653), wherein, in an abutting position of the lever (653), the friction wheel (652) abuts against the operating wheel (13) of the endoscope (1) connected to the rotatable assembly (60), and wherein the lever (653) is pivotable by means of an actuating element (656, 656').

9. Endoscope device according to the preceding claim, wherein the actuating element comprises a handwheel (656') rotatable about the longitudinal axis (L), which handwheel is rotatably arranged at the rotatable assembly (60) and movable for moving the friction wheel (652) from the abutting position.

10. Endoscope device according to one of the preceding claims, wherein the first drive means (63) is embodied to drive a first rotatable assembly (60B), which is mechanically connectable to the endoscope (1), and wherein the second drive means (64) arranged at the stationary assembly (61) is embodied to drive a second rotatable assembly (60A), which is embodied coupleable to the operating wheel (13) of the endoscope (1).

11. Endoscope device according to the preceding claim, wherein the first and the second rotatable assembly (60A, 60B) respectively comprise a ring element (603A, 603B) rotatable about the longitudinal axis (L) relative to the stationary assembly (61), which ring element is engaged via a toothing (617A, 617B) to a pinion (631', 641) of the respectively assigned drive means (63, 64) and embodied to transmit a torque to the endoscope (1) or to the operating wheel (13) of the endoscope (1).

12. Endoscope device according to one of the preceding claims, wherein the rotational device (6) furthermore comprising a light source (15) for generating and feeding light into the endoscope (1).

13. Endoscope device according to the preceding claim, wherein the light source (15) is arranged at the rotatable assembly (60) and embodied to rotate with the rotatable assembly (60).

## Revendications

1. Dispositif d'endoscope, comprenant :
un endoscope (1) qui présente une tige (10) et une molette de commande (13) pouvant tourner autour de l'axe longitudinal (L) de l'endoscope (1) pour déplacer un module déplaçable (11) de l'endoscope (1) pour faire varier une direction de visée de l'endoscope (1) en déplaçant un élément optique (11) ou pour faire varier un ajustement de focalisation ou de grossissement d'un système optique de l'endoscope (1) ; et
un dispositif de rotation (6) pour faire tourner l'endoscope (1), le dispositif de rotation (6) comprenant
un module rotatif (60) avec lequel l'endoscope (1) peut être connecté ;
un module fixe (61) par rapport auquel le module rotatif (60) peut tourner ;
un premier dispositif d'entraînement (63) pour faire tourner le module rotatif (60) par rapport au module fixe (61), afin d'animer d'un mouvement de rotation autour de l'axe longitudinal (L) de l'endoscope (1) la tige (10) de l'endoscope (1) connecté au module rotatif (60) ;
un deuxième dispositif d'entraînement (64) pour faire tourner la molette de commande (13) de l'endoscope (1), pouvant tourner autour de l'axe longitudinal de l'endoscope (1) connecté au module rotatif (60) par rapport à la tige (10) de l'endoscope (1) afin de déplacer un module (11) de l'endoscope (1) déplaçable au moyen de la molette de commande (13), le deuxième dispositif d'entraînement (64) étant réalisé pour faire tourner la molette de commande (13) de l'endoscope (1) autour de l'axe longitudinal (L) de l'endoscope (1).

2. Dispositif d'endoscope selon la revendication 1, dans lequel le deuxième dispositif d'entraînement (64) est réalisé pour provoquer, par actionnement de la molette de commande (13), une modification d'une direction de visée de l'endoscope (1) par déplacement d'un élément optique (11) ou une modification d'un ajustement de focalisation ou de grossissement d'un système optique de l'endoscope (1).

3. Dispositif d'endoscope selon la revendication 1 ou 2, dans lequel le deuxième dispositif d'entraînement (64) est disposé au niveau du module rotatif (60) ou au niveau du module fixe (61).

4. Dispositif d'endoscope selon l'une quelconque des revendications précédentes, dans lequel le dispositif de rotation (6) comprend en outre :
un adaptateur (62, 606B) ou un dispositif de serrage (621', 622', 605B) pour l'accouplement solidaire en rotation du module rotatif (60) à l'endoscope (1).

5. Dispositif d'endoscope selon la revendication précédente, dans lequel l'adaptateur (62) peut être retiré du module rotatif (60) le long de l'axe longitudinal (L) afin de séparer l'endoscope (1) du module rotatif (60), ou peut être appliqué contre le module rotatif (60) le long de l'axe longitudinal (L) afin d'accoupler mécaniquement l'endoscope (1) au module rotatif (60).

6. Dispositif d'endoscope selon la revendication 4 ou 5, dans lequel l'adaptateur (62) présente deux parties d'adaptateur déplaçables l'une par rapport à l'autre (625, 626) qui sont réalisées de manière à recevoir entre elles l'endoscope (1) de manière solidaire en rotation.

7. Dispositif d'endoscope selon l'une quelconque des revendications 4 à 6, dans lequel l'adaptateur (62), dans un état connecté au module rotatif (60), est accouplé au module rotatif (60) de manière appropriée pour le transfert d'un couple autour de l'axe longitudinal (L) et pour le transfert d'une force parallèlement à l'axe longitudinal (L).

8. Dispositif d'endoscope selon l'une quelconque des revendications précédentes, dans lequel le dispositif de rotation (6) comprend en outre :
un dispositif de transfert (641, 652) qui est accouplé au deuxième dispositif d'entraînement (64) afin d'exercer, sous l'effet de l'entraînement par le deuxième dispositif d'entraînement (64), une force de réglage sur la molette de commande (13), le dispositif de transfert comprenant une roue de friction (652) qui est disposée au niveau d'un bras de levier d'un levier pivotant (653), la roue de friction (652), dans une position d'appui du levier (653), étant en appui contre la molette de commande (13) de l'endoscope (1) connecté au module rotatif (60) et le levier (653) pouvant pivoter au moyen d'un élément d'actionnement (656, 656').

9. Dispositif d'endoscope selon la revendication précédente, dans lequel l'élément d'actionnement comprend une roue à main (656') pouvant tourner autour de l'axe longitudinal (L), qui est disposée de manière rotative sur le module rotatif (60) et qui peut être déplacée hors de la position d'appui pour déplacer la roue de friction (652).

10. Dispositif d'endoscope selon l'une quelconque des revendications précédentes, dans lequel le premier dispositif d'entraînement (63) est réalisé pour l'entraînement d'un premier module rotatif (60B) qui peut être connecté mécaniquement à l'endoscope (1) et dans lequel le deuxième dispositif d'entraînement (64) disposé au niveau du module fixe (61) est réalisé pour l'entraînement d'un deuxième module rotatif (60A) qui peut être accouplé à la molette de commande (13) de l'endoscope (1).

11. Dispositif d'endoscope selon la revendication précédente, dans lequel le premier et le deuxième module rotatif (60A, 60B) présentent chacun un élément annulaire (603A, 603B) pouvant tourner autour de l'axe longitudinal (L) par rapport au module fixe (61), qui est en prise par le biais d'une denture (617A, 617B) avec un pignon (631', 641) du dispositif d'entraînement respectivement associé (63, 64) et qui est réalisé pour transférer un couple à l'endoscope (1) ou à la molette de commande (13) de l'endoscope (1).

12. Dispositif d'endoscope selon l'une quelconque des revendications précédentes, dans lequel le dispositif de rotation (6) comprend en outre une source de lumière (15) pour générer et injecter de la lumière dans l'endoscope (1).

13. Dispositif d'endoscope selon la revendication précédente, dans lequel la source de lumière (15) est disposée et réalisée au niveau du module rotatif (60) afin de tourner avec le module rotatif (60).
